(19) Europäisches Patentamt / European Patent Office / Office européen des brevets

(11) **EP 1 017 390 B1**

(12) **EUROPEAN PATENT SPECIFICATION**

(45) Date of publication and mention
of the grant of the patent:
**18.04.2007 Bulletin 2007/16**

(51) Int Cl.:
*A61K 31/455* (2006.01)    *A61K 31/785* (2006.01)
*A61K 45/06* (2006.01)     *A61K 9/20* (2006.01)

(21) Application number: **98938228.8**

(22) Date of filing: **31.07.1998**

(86) International application number:
**PCT/US1998/015990**

(87) International publication number:
**WO 1999/006035 (11.02.1999 Gazette 1999/06)**

(54) **COATED TABLET COMPRISING NICOTINIC ACID OR A COMPOUND METABOLIZED TO NICOTINIC ACID IN AN EXTENDED RELEASE FORM, AND A COATING CONTAINING AN HMG-COA REDUCTASE INHIBITOR IN AN IMMEDIATE RELEASE FORM**

MANTELTABLETTE ENTHALTEND NIKOTINSÄURE ODER EINEN STOFF, DER ZU NIKOTINSÄURE METABOLISIERT WIRD, IN VERZÖGERT FREIGESETZTER FORM, UND EINEN HMG-COA REDUKTASEHEMMER IM MANTEL ZUR SOFORTIGEN FREISETZUNG

COMPRIME ENROBE COMPRENANT DE L'ACIDE NICOTINIQUE OU UN COMPOSE METABOLISE EN ACIDE NICOTINIQUE A LIBERATION RETARDEE, ET D'INHIBITEUR DE REDUCTASE HMG-CoA A LIBERATION IMMEDIATE

(84) Designated Contracting States:
**AT BE CH CY DE DK ES FI FR GB GR IE IT LI LU MC NL PT SE**

(30) Priority: **31.07.1997 US 903752**

(43) Date of publication of application:
**12.07.2000 Bulletin 2000/28**

(60) Divisional application:
**07003276.8**

(73) Proprietor: **Kos Life Sciences, Inc.**
**Weston, FL 33326 (US)**

(72) Inventors:
• **BOVA, David J.**
**Miami Lakes, FL 33014 (US)**
• **DUNNE, Josephine**
**Miami, FL 33020 (US)**

(74) Representative: **Findlay, Alice Rosemary et al**
**Lloyd Wise**
**Commonwealth House,**
**1-19 New Oxford Street**
**London WC1A 1LW (GB)**

(56) References cited:
EP-A- 0 401 705         EP-A- 0 409 181
EP-A- 0 577 504         EP-A- 0 671 171
WO-A-96/32942           US-A- 4 933 165
US-A- 5 135 935         US-A- 5 260 305
US-A- 5 427 799

• ERKELENS: "Combination Drug Therapy with HMG CoA Reductase Inhibitors and Bile Acid Sequestrants for Hypercholesteremia" CARDIOLOGY, vol. 77, no. SUPPL. 04, 1990, pages 33-38, XP002084817
• PATENT ABSTRACTS OF JAPAN vol. 013, no. 156 (C-585), 14 April 1989 & JP 63 310827 A (SANWA KAGAKU KENKYUSHO CO LTD), 19 December 1988

Remarks:
The file contains technical information submitted after the application was filed and not included in this specification

EP 1 017 390 B1

**Description**

**Field of the Invention**

This invention relates to a coated tablet for oral administration comprising nicotinic acid or a nicotinic acid compound or mixtures thereof in an extended release form and a coating containing a 3-hydroxy-3-methylglutaryl co-enzyme A (HMG-CoA) reductase inhibitor in an immediate release form which is useful for altering serum lipid levels in subjects when given once per day as a single dose during the evening hours, without causing drug-induced hepatotoxicity, myopathy or rhabdomyolysis.

**Background**

Hyperlipidemia or an elevation in serum lipids is associated with an increase incidence of cardiovascular disease and atherosclerosis. Specific forms of hyperlipidemia include, for example, hypercholesteremia, familial dysbetalipoproteinemia, diabetic dyslipidemia, nephrotic dyslipidemia and familial combined hyperlipidemia. Hypercholesteremia is characterized by an elevation in serum low density lipoprotein-cholesterol and serum total cholesterol. Low density lipoprotein (LDL-cholesterol) transports cholesterol in the blood. Familial dysbetalipoproteinemia, also known as Type III hyperlipidemia, is characterized by an accumulation of very low density lipoprotein-cholesterol (VLDL-cholesterol) particles called beta-VLDLs in the serum. Also associated with this condition, there is a replacement of normal apolipoprotein E3 with abnormal isoform apolipoprotein E2. Diabetic dyslipidemia is characterized by multiple lipoprotein abnormalities, such as an overproduction of VLDL-cholesterol, abnormal VLDL triglyceride lipolysis, reduced LDL-cholesterol receptor activity and, on occasion. Type III hyperlipidemia. Nephrotic dyslipidemia is difficult to treat and frequently includes hypercholesteremia and hypertriglyceridemia. Familial combined hyperlipidemia is characterized by multiple phenotypes of hyperlipidemia. i.e., Type IIa. IIb, IV, V or hyperapobetalipoproteinemia.

It is well known that the likelihood of cardiovascular disease can be decreased, if the serum lipids, and in particular LDL-cholesterol can be reduced. It is also well known that the progression of atherosclerosis can be retarded or the regression of atherosclerosis can be induced if serum lipids can be lowered. In such cases, individuals diagnosed with hyperlipidemia or hypercholesteremia should consider lipid-lowering therapy to retard the progression or induce the regression of atherosclerosis for purposes of reducing their risk of cardiovascular disease, and in particular coronary artery disease.

Hypertriglyceridemia is also an independent risk factor for cardiovascular disease, such as coronary artery disease. Many people with hyperlipidemia or hypercholesteremia also have elevated triglyceride levels. It is known that a reduction in elevated triglycerides can result in the secondary lowering of cholesterol. These individuals should also consider lipid-lowering therapy to reduce their elevated triglycerides for purposes of decreasing their incidence of atherosclerosis and coronary artery disease.

Cholesterol is transported in the blood by lipoprotein complexes, such as VLDL-cholesterol. LDL-cholesterol, and high density lipoprotein-cholesterol (HDL-cholesterol). LDL carries cholesterol in the blood to the subendothelial spaces of blood vessel walls. It is believed that peroxidation of LDL-cholesterol within the subendothelial space of blood vessel walls leads to atherosclerosis plaque formation. HDL-cholesterol, on the other hand, is believed to counter plaque formation and delay or prevent the onset of cardiovascular disease and atherosclerotic symptoms. Several subtypes of HDL-cholesterol, such as $HDL_1$-cholesterol, $HDL_2$-cholesterol and $HDL_3$-cholesterol, have been identified to date.

In the past, there have been numerous methods proposed for reducing elevated cholesterol levels and for increasing HDL-cholesterol levels. Typically, these methods include diet and/or daily administration of lipid-altering or hypolipidemic agents. Another method proposed concerns periodic plasma dilapidation by a continuous flow filtration system, as described in U.S. Patent No. 4,895,558. Several types of hypolipidemic agents have been developed to treat hyperlipidemia or hypercholesteremia or normolipidemics diagnosed with cardiovascular disease. In general, these agents act (1) by reducing the production of the serum lipoproteins or lipids, or (2) by enhancing their removal from the serum or plasma. Drugs that lower the concentration of serum lipoproteins or lipids include inhibitors of HMG-CoA reductase, the rate controlling enzyme in the biosynthetic pathway of cholesterol. Examples of HMG-CoA reductase inhibitors include mevastatin, U.S. Patent No. 3,983,140, lovastatin also referred to as mevinolin, U.S. Patent No 4,231,938, pravastatin, U.S. Patent Nos. 4,346,227 and 4,410,629. lactones of pravastatin, U.S. Patent No. 4,448,979, velostatin, also referred to as synvinolin, simvastatin, U.S. Patent Nos. 4,448,784 and 4,450,171, rivastatin, fluvastatin, atorvastatin and cerivastatin. For other examples of HMG-CoA reductase inhibitors, see U.S. Patent Nos. 3,217,992 ; 5,196,440; 5.189,180; 5,166,364; 5,157,134; 5,110,940; 5,106;992: 5,099,035;5,081,136;5,049,696;5,049,577; 5,025,017; 5,011,947; 5.010,105; 4,970,221; 4,940,800; 4,866,058; 4,686,237; 4.647,576; European Application Nos. 0142146A2 and 0221025A1: and PCT Application Nos. WO 86/03488 and WO 86/07054.

Other drugs which lower serum cholesterol include, for example, nicotinic acid, bile acid sequestrants, e.g., cholestyramine, colestipol DEAESephadex (Secholex® and Polidexide®), probucol and related compounds as disclosed in U.S. Patent No. 3.674.836. lipostabil (Rhone-Poulanc). Eisai E5050 (an N-substituted ethanolamine derivative), imanixil (HOE-402) tetrahydrolipstatin (THL), isitigmastanyl phosphorylcholine (SPC. Roche), aminocyclodextrin (Tanabe Seiyoku), Ajinomoto AJ-814 (azulene derivative), melinamide (Sumitomo), Sandoz 58-035. American Cyanimid CL-277,082 and CL-283,546 (disubstituted urea derivatives), ronitol (which has an alcohol which corresponds to nicotinic acid), neomycin, p-aminosalicylic acid, aspirin, quarternary amine poly(diallyldimethylammonium chloride) and ionenes such as disclosed in U.S. Patent No. 4,027,009, poly(diallylmethylamine) derivatives such as disclosed in U.S. Patent No. 4,759,923. omega-3-fatty acids found in various fish oil supplements, fibric acid derivatives, e.g., gemfibrozil, clofibrate, bezatibrate, fenofibrate, ciprofibrate and clinotibrate, and other known serum cholesterol lowering agents such as those described in U.S. Patent No. 5,200,424; European Patent Application No. 0065835A1, European Patent No, 164-698-A.G.B. Patent No. 1,586,152 and G.B. Patent Application No. 2162-179-A.

Nicotinic acid, also known as niacin, has been used for many years in the treatment of hyperlipidemia or hypercholesteremia. This compound has long been known to exhibit the beneficial effects of reducing total cholesterol, VLDL-cholesterol and VLDL-cholesterol remnants, LDL-cholesterol, triglycerides and apolipoprotein a, known as "Lp (a)," in the human body, while increasing desirable HDL-cholesterol.

Nicotinic acid has normally been administered three times per day after meals. This dosing regimen is known to provide a very beneficial effect on blood lipids as discussed in Knopp et al.; "Contrasting Effects of Unmodified and Time-Release Forms of Niacin on Lipoproteins in Hyperlipidemic Subjects: Clues to Mechanism of Action of Niacin"; Metabolism (34)7:642-647 (1985). The chief advantage of this profile is the ability of nicotinic acid to decrease total cholesterol. LDL-cholesterol, triglycerides and $L_p(a)$ while increasing HDL-cholesterol particles. While such a regimen does produce beneficial effects, cutaneous flushing and the like still often occurs in the hyperlipidemics to whom the nicotinic acid is administered.

In order to avoid or reduce the cutaneous flushing resulting from nicotinic acid therapy, a number of agents have been suggested for administration with an effective antihyperlipidemic amount of nicotinic acid, such as guar gum as reported in U.S. Patent No. 4,965,252, mineral salts as disclosed in U.S. Patent No. 5,023,245, inorganic magnesium salts as reported in U.S. Patent No. 4,911,917, and non-steroidal anti-inflammatories, such as aspirin, as disclosed in PCT Application No. 96/32942. These agents have been reported to avoid or reduce the cutaneous flushing side effect commonly associated with nicotinic acid dividend dose treatment.

Another method of avoiding or reducing the side effects associated with immediate release niacin is the use of extended or sustained release formulations. Extended or sustained release formulations are designed to slowly release the active ingredient from the tablet or capsule, which allows a reduction in dosing frequency as compared to the typical dosing frequency associated with conventional or immediate dosage forms. The slow drug release reduces and prolongs blood levels of the drug and, thus, minimizes or lessens the cutaneous flushing side effects that are associated with conventional or immediate release niacin products. Extended or sustained release formulations of niacin have been developed, such as Nicobid® capsules (Rhone-Poulenc Rorer), Endur-acin® (Innovite Corporation),and the formulations described in U.S. Patent. Nos. 5,126,145 and 5.268.181. which describe a sustained release niacin formulation containing two different types of hydroxy propyl methylcelluloses and a hydrophobic component.

Studies in hyperlipidemic patients have been conducted with a number of extended or sustained release niacin products. These studies have demonstrated that the extended or sustained release products do not have the same advantageous lipid-altering effects as immediate release niacin, and in fact have a worse side effect profile compared to the immediate release product. The major disadvantage of the sustained release formulations, as reported in Knopp et al.: Metabolism, 34(7):642-647 (1985), is the significantly lower reduction in triglycerides (-2% for the sustained release versus - 38% for the immediate release) and lower increase in HDL-cholesterol (+8% for the sustained release versus +22% for the immediate release) and $HDL_2$-cholesterol particles, which are known by the art to be most beneficial (-5% for the sustained release versus +37% for the immediate release).

Additionally, extended or sustained release niacin formulations are known to cause greater incidences of liver toxicity, as described in Henken et al.: Am J Med, 91:1991 (1991) and Dalton et al.: Am J Med, 93: 102 (1992). There is also great concern regarding the potential of these formulations in disrupting glucose metabolism and uric acid levels.

In a previous edition of the Journal of the American Medical Association (JAMA), an article appeared which presented research results investigating the liver toxicity problems associated with a sustained release form of nicotinic acid. "A Comparison of the Efficacy and Toxic Effects of Sustained- vs. Immediate-Release Niacin in Hypercholesterolemic Patients", McKenney et al., JAMA, 271 (9): 672 (March 2. 1994). The article presented a study of twenty-three patients. Of that number, 18 or 78 percent were forced to withdraw because liver function tests (LFTs) increased indicating potential liver damage. The conclusion of the authors of that article was that the sustained release form of niacin "should be restricted from use."

A similar conclusion was reached in an article by representatives of the Food and Drug Administration and entitled "Hepatic Toxicity of Unmodified and Time-Release Preparations of Niacin", Rader et al.: Am J Med, 92:77 (January, 1992). Because of these studies and similar conclusions drawn by other health care professionals, the sustained release forms of niacin have experienced limited utilization.

HMG-CoA reductase inhibitors have also been used for many years to treat hyperlipidemia. These compounds are known to exhibit beneficial effects of reducing total cholesterol and LDL-cholesterol in the human body, and elevating HDL-cholesterol levels in some individuals. Grundy SM: N Engl J Med, 319(1):24-32, at 25-26 and 31 (July 7, 1988). The conversion of HMG-CoA to mevalonate is an early step in the biosynthesis of cholesterol. Inhibition of HMG-CoA reductase, which interferes with the production of mevalonate is the basis by which the HMG-CoA reductase inhibitors exert their total cholesterol-loweringand LDL-cholesterol-loweringeffects. Grundy SM: N Engl J Med, 319(1):24-32, at 25 and 26 (July 7. 1988).

HMG-CoA reductase inhibitors are not without drawback, however. HMG-CoA reductase inhibitors are known to induce hepatotoxicity, myopathy and rhabdomyolysis, as reported in, for example. Garnett WR: Am J Cardiol, 78 (Suppl 6A):20-25 (Sept. 26. 1996); The Lovastatin Pravastatin Study Group: Am J Cardiol. 71:810-815 (April 1. 1993); Dujovne CA et al.: Am J Med, 91 (Suppl 1B):25S-30S (July 31. 1991), and Mantell GM et al.: Am J Cardiol, 66:11B-15B (Set. 18. 1990).

Moreover, on Page 1700, in column 3. of the Physicians' Desk Reference (PDR) 50th Ed., 1996, it reports that lovastatin, an HMG-CoA reductase inhibitor should be used with caution in patients who have a past history of liver disease, and that lovastatin therapy is contraindicated for those individuals with active liver disease or unexplained persistent elevations of serum transaminases. The 1996 PDR further reports on Page 1701, in column 1, that rhabdomyolysis has been associated with lovastatin therapy alone and when combined with lipid-lowering doses ($\geq$ 1g/day) of nicotinic acid, and that physicians contemplating combined therapy with lovastatin and lipid-lowering doses of nicotinic acid should carefully weigh the potential benefits and risks and should carefully monitor individuals for any signs and symptoms of muscle pain, tenderness, or weakness; particularly during the initial months of therapy and during any periods of upward dosage titration of either drug. The 1996 PDR further reports on page 1701, in column 1, that cases of myopathy have been associated with patients taking lovastatin concomitantly with lipid-lowering doses of nicotinic acid. The 1996 PDR also reports similar contraindications (1) for fluvastatin on page 2267, column 3, and on page 2268, column 1, (2) for pravastatin on page 767. column 1, and (3) for simvastatin on page 1777, column 2. Still further, the PDR recommends on page 768, column 3, that concomitant therapy with HMG-CoA reductase inhibitors and these agents [lipid lowering doses of nicotinic acid] is generally not recommended.

Notwithstanding the recommendations in the 1996 PDR, Grundy SM: N Engl J Med, 319(1):24-33 (July 7, 1988), reports that HMG-CoA reductase inhibitors when used alone (at pages 29-30) and nicotinic acid when used alone (at page 24) are effective in reducing elevated cholesterol plasma levels. Grundy further reports on page 24. in column 2 at lines 10-25. that "[b]ecause of their efficacy...bile acid sequestrants (cholestyramine and colestipol) and niacin are probably the drugs of first choice for hypercholesteremia...Although these drugs can be highly effective and are satisfactory for use in many patients with high cholesterol levels, they unfortunately are not well tolerated by all patients. Therefore, in spite of their proved usefulness, bile acid sequestrants and niacin are not ideal cholesterol-lowering agents." Still further. Grundy reports on page 30, in column I at lines 13-17, that the "...administrationof [HMG-CoA] reductase inhibitors twice a day is somewhat more effective than administration once a day, at the same total dosage." Grundy also reports on page 29, in column 1 at lines 7-11. "...that the combination of lovastatin and cyclosporine, gemfibrozil or nicotinic acid may predispose patients to myopathy and occasionally even to rhabdomyolysis." Still further, Grundy reports on page 30, in column 1 at lines 54-59, that "[the combination of lovastatin and niacin has not been shown to be safe in a controlled clinical trial; furthermore, a manifestation of an adverse interaction between the agents, such as myopathy, could occur." But see Gardner SF et al.: Pharmacotherapy. 16(3):421-423 (1996): Pasternak RC et al.: Ann Intern Med, 125(7):529-540(Oct. 1. 1996); O'Keefe JH et al.: Am J Cardiol, 76:480-484 (Sept. 1,995); and Davignon J et al.: Am J Cardiol, 73:339-345 (Feb. 15, 1994).

In Vacek JL et al.: Am J Cardiol, 76:182-184 (July. 15, 1995), they report on page 183 that "...because of the present state of knowledge of the risks of hepatotoxicity with slow-release forms of nicotinic acid, this form of the drug should probably not be used [in combination with lovastatin] in future trials or clinical practice."

Consistent with the reports by Vacek JL et al. and the 1996 PDR, the article by Jacobson TA and Amorosa LF: Am J Cardiol, 73:25D-29D (May 26, 1994), reports, on pages 28D-29D, that because "[a]bnormalities in liver enzyme profiles and fulminant hepatic failure have also been associated with the use of niacin, particularly sustained-release preparations...the use of fluvastatin in combination with a sustained release niacin preparation cannot generally be recommended based upon this study, which only examined crystalline or immediate release niacin."

Therefore, it can be seen from the scientific literature that there is a need for development of lipid-altering or hypolipidemic pharmaceuticals and methods of delivering said pharmaceuticals which would provide patients with "balanced lipid alteration." i.e.. reductions in total cholesterol. LDL- cholesterol, triglycerides and Lp(a), as well as increases in HDL particles, with an acceptable safety profile, especially as to liver toxicity, effects on glucose metabolism, uric acid levels, myopathy and rhabdomyolysis.

US 5,260,350 discloses a pharmaceutical combination of an HMg-CoA reductase inhibitor and nicotinic acid or a related acid. The combination is useful for reducing serum cholesterol and triglycerides and preventing or treating atherosclerosis without causing drug-induced myopathy or rhabdomyolysis.

## Summary of the Invention

In brief, the present invention alleviates and overcomes certain of the above-identified problems and shortcomings of the present state of HMG-CoA reductase inhibitor therapy and nicotinic acid therapy through the discovery of a novel HMG-CoA reductase/nicotinic acid pharmaceutical combination for oral administration which is defined in the claims.

In accordance with the present invention, a pharmaceutical combination for oral administration is provided to alter serum lipid levels in individuals, e.g.. reducing hyperlipidemia and inhibiting atherosclerosis, without causing drug-induced hepatoxicity, rhabdomyolysis, or myopathy. Generally speaking, the pharmaceutical combinations of the present invention comprise nicotinic acid, a derivative of nicotinic acid, a compound which is metabolized by the body to form nicotinic acid or any mixtures thereof in an extended release form, and an HMG-CoA reductase inhibitor. The pharmaceutical combinations are administered in amounts which are effective to alter or reduce serum lipids levels such as total cholesterol. VLDL-cholesterol. LDL-cholesterol, Lp(a) and triglycerides levels, and to enhance or increase HDL-cholesterol levels. This is accomplished without causing drug-induced hepatotoxicity, rhabdomyolysis or myopathy or adversely effecting glucose metabolism or uric acid levels, or at least without causing such side effects in at least an appreciable number of individuals to such a level that discontinuation of such therapy would be required.

In accordance with the present invention, the pharmaceutical combinations are administered once a day as a single oral dose. Preferably, and for those individuals on a typical day time schedule, the single oral dose is administered during evening hours, such as with or after their evening meals or at their bedtimes, to achieve in those individuals during the night effective in vivo levels for reducing total cholesterol, VLDL-cholesterol. LDL-cholesterol. Lp(a) and triglycerides levels and for enhancing or increasing HDL-cholesterol levels, some of which lipid components are biosynthesized predominantly at night in such individuals. For those individuals with typical night time, as opposed to day time, schedules, e.g., those individuals who work through the night and sleep during the day, it may be preferable to administer the pharmaceutical combinations of the present invention as a single oral dose at or near their day time bedtimes.

It also has been found that, when a pharmaceutical combination of the present invention is administered once a day as a single oral dose, the single dose provides additional total cholesterol. LDL-cholesterol, and triglyceride reduction effects over that which is obtained using the nicotinic acid alone. In fact, it has been found that the pharmaceutical combinations of the present invention, when administered as a single oral dose, reduces total cholesterol. LDL-cholesterol and triglycerides levels to a substantially greater extent than when either lipid-loweringdrug is administered alone as a single oral dose in an equal dosage amount. Moreover, it has been found that the pharmaceutical combinations of the present invention, when administered as a single oral dose, increases HDL-cholesterol levels to a substantially greater extent than when the HMG-CoA reductase inhibitor is administered alone as a single oral dose in an equal dosage amount. It is also believed that, when the pharmaceutical combinations of the present invention are administered once a day as a single dose, the single oral dose (1) is at least as effective as the combination of an equal or higher daily dosage of nicotinic acid administered in divided oral doses and an equal daily oral dosage of HMG-CoA reductase inhibitor administered separate from the divided doses of nicotinic acid, and (2) it has less capacity to provoke hepatotoxicity than the divided dose therapy.

Quite surprisingly, the pharmaceutical combinations of the present invention can be used to effectively treat, for instance, hyperlipidemia (e.g., cholesterol-related cardiovascular disease) and atherosclerosis of multiple etiology, and normolipidemics diagnosed with or predisposed to cardiovascular disease, without causing drug-induced liver damage, rhabdomyolysis or myopathy, or adversely effecting glucose metabolism or uric acid levels.

The pharmaceutical combinations of the present invention contemplate the combination of (a) an HMG-CoA reductase inhibitor in an immediate release form and (b) nicotinic acid, as well as derivatives of nicotinic acid, compounds which the body metabolizes to nicotinic acid and any combinations thereof in an extended release form.

Preferred HMG-CoA reductase inhibitors include atorvastatin, cervastatin, fluvastatin, lovastatin, pravastatin and simvastatin.

In carrying out a method of the present invention, the pharmaceutical combinations of the present invention can be administered to humans and other animal species, such as bovines, canines, felines, porcines, equines, sheep, rabbits: mice, rats, rodents, monkeys, etc. Other lipid-alteringor hypolipidemicagents as well as agents known to reduce or prevent cutaneous flushing may be included in the pharmaceutical combinations or administered concomitantly with the pharmaceutical combinations in appropriate regimens which complement the beneficial effects of the pharmaceutical combinations of the present invention, so long as such additives do not defeat the objectives of the present invention.

The present invention also contemplates pretreating subjects with a nonsteroidal anti-inflammatory drug (NSAID) prior to the start of nicotinic acid therapy to reduce or eliminate nicotinic acid induced flushing which limits patient compliance. Pretreatment with low dosages of an NSAID, such as aspirin, when used according to a predosing schedule, cumulatively suppresses prostaglandin $D_2$ ($PGD_2$) production, making administration of nicotinic acid more tolerable. In accordance with the present invention, predosing a subject with an NSAID involves administering a low dose NSAID, such as aspirin, one to four times a day for at least about 7 days, and preferably for at least about 14 days, prior to nicotinic acid administration.

The doses administered should be carefully adjusted according to age, weight and condition of the patient, as well as the route of administration, dosage form and regimen and the desired result.

Thus, for oral administration, a satisfactory result may be obtained employing an HMG-CoA reductase inhibitor in dosages as indicated in, for example, the 1996 Physician's Desk Reference or package inserts for those products, such as in an amount within the range of from about 0.05mg to about 160mg, and preferably from about 0.05 to 80mg, and more preferably from about 0.2mg to about 40mg, in combination with nicotinic acid in dosages normally employed, as indicated in the 1996 Physician's Desk Reference, for nicotinic acid, such as in an amount within the range of from about 250mg to about 3000mg, and preferably from about 500mg to about 2500mg, and most preferably from about 1000mg to about 2000mg, with the HMG-CoA reductase inhibitor and nicotinic acid being employed together in the same oral dosage form or in separate oral dosage forms taken at the same or about the same time. The nicotinic acid, therefore, may be daily dosed in increments of, for example, 250mg, 500mg, 750mg, 1000mg, 1500mg, 2000mg, 2500mg and 3000mg. Thus, the oral dosage forms of the present invention may include nicotinic acid in dosage amounts of for example. 250mg, 375mg, 500mg, 750mg and 1000mg.

It should be understood to those versed in this art that the exact dosing for an HMG-CoA reductase inhibitor will depend upon the particular HMG-CoA reductase inhibitor selected. Therefore, and in accordance with the present invention, the oral dosage forms may include lovastatin, atorvastatin or pravastatin in dosage amounts of, for example, between about 10mg and about 80mg or more, such as 10mg, 20mg. 40mg or 80mg, simvastatin in dosage amounts of, for example, between about 5mg and about 80mg or more, such as 5mg. 10mg. 20mg, 40mg or 80mg, fluvastatin in dosage amounts of, for example, between about 20mg and 80mg or more, such as 20mg, 40mg or 80mg, and cerivastatin in dosage amounts of for example, between about 0.05mg and about 0.3mg or more, such as 0.5mg. 0.1mg, 0.2mg and 0.3mg, to achieve a desired daily dosage.

Thus, and in accordance with the present invention, an oral solid dosage form, such as tablets, may contain the HMG-CoA reductase inhibitor in an amount of from about 0.05mg to about 40mg, and preferably from about 0.1mg to about 20mg, and nicotinic acid in an amount of from about 250mg to about 1000mg, and preferably from 500mg to about 1000mg. Examples of oral solid dosage forms in accordance with the present invention include: nicotinic acid/ atorvastatin, fluvastatin, lovastatin, pravastatin, or simvastatin tablets in dosage strengths of, for instance, 250mg/5mg, 500mg/5mg, 750mg/5mg, 1000mg/5mg, 250mg/7.5mg, 500mg/7.5mg, 750mg/7.5mg, 1000mg/7.5mg, 250mg/10mg, 500mg/10mg, 750mg/10mg, 1000mg/10mg, 250mg/20mg, 500mg/20mg, 750mg/20mg, 1000mg/20mg tablets, 250mg/ 40mg, 500mg/40mg, 750mg/40mg, and 1000mg/40mg; and nicotinic acid/cerivastatin tablets in dosage strengths of for instance. 250mg/0.05mg. 500mg/0.05mg, 750mg/0.05mg, 1000mg/0.05mg. 250mg/0.1mg. 500mg/0.1mg, 750mg/ 0.1mg, 1000mg,/0.1mg, 250mg/0.15mg, 500mg/0.15mg. 750mg/0.15mg, 1000mg/0.15mg tablets, 250mg/0.2mg. 500mg/0.2mg, 750mg/0.2mg. 1000mg/0.2mg tablets, 250mg/0.3mg. 500mg/0.3mg, 750mg/0.3mg and 1000mg/0.3mg tablets.

It is therefore an object of the present invention to provide a pharmaceutical combination for oral administration comprising (a) an HMG-CoA reductase inhibitor, and (b) nicotinic acid, derivatives of nicotinic acid, compounds which are metabolized by the body to form nicotinic acid and combinations thereof in a sustained release form for altering serum lipids to treat subjects, e.g.. subjects diagnosed with hyperlipidemia, atherosclerosis and lipidemia in normolipidemics.

It is another object of the present invention to provide an oral solid pharmaceutical combination having extended release characteristics for the nicotinic acid, a derivative of nicotinic acid, a compound metabolized to nicotinic acid by the body or mixtures thereof, and having immediate release characteristics for the HMG-CoA reductase inhibitor.

In general, the present invention provides an improved iipid-atterins or antihyperlipidemia pharmaceutical combination of the oral type employing an effective lipid-altering or antihyperlipidemic amount of an HMG-CoA reductase inhibitor and nicotinic acid, wherein the pharmaceutical combination comprises compounding the nicotinic acid with, for example, from about 5% to about 50% parts by weight of hydroxy propyl methyl cellulose per hundred parts by weight of the tablet or formulation and coating the tablet with an HMG-CoA reductase inhibitor from about 0.01% to about 30% parts by weight of the tablet or formula.

The present invention also provides an orally administered lipid altering or antihyperlipidemiacomposition which comprises from about 0.01% to about 30% parts by weight of an HMG-CoA reductase inhibitor; from about 30% to about 90% parts by weight of nicotinic acid; and, from about 5% to about 50% parts by weight of hydroxy propyl methyl cellulose.

A method of treating hyperlipidemia in a hyperlipidemic or lipidemia in a normolipidemic comprises dosing the hyperlipidemicor normolipidemic with an effective lipid-altering amount of an HMG-CoA reductase inhibitor and nicotinic acid, a derivative of nicotinic acid, a compound metabolized to nicotinic acid by the body or mixtures thereof. The dose is given once per day, preferably in the evening or at night, combined with a pharmaceutically acceptable carrier to produce a significant reduction in total cholesterol and LDL-cholesterol as well as a significant reduction in triglycerides and Lp(a), with a significant increase in HDL cholesterol.

The above features and advantages of the present invention will be better understood with the reference to the following detailed description and examples.

**Detailed Description**

By way of illustrating and providing a more complete appreciation of the present invention and many of the attendant advantages thereof, the following detailed description and examples are given concerning the novel methods and pharmaceuticals.

The present invention employs an HMG-CoA reductase inhibitor and nicotinic acid, a derivative of nicotinic acid or a compound other than nicotinic acid itself which the body metabolizes into nicotinic acid and mixtures thereof, thus producing the same effect as described herein. The nicotinic acid derivatives and other compounds specifically include the following: nicotinyl alcohol tartrate, d-glucitol hexanicotinate, aluminum nicotinate, niceritrol, d,1-alpha-tocopheryl nicotinate, 6-OH-nicotinic acid, nicotinaria acid, nicotinamide, nicotinamide-N-oxide. 6-OH-nicotinamide,NAD, N-methyl-2-pyrridine-8-carboxamide N-methyl-nicotinamide, N-ribosyl-2-pyridone-5-carboxide, N-methyl-4-pyridone-5-carboxamide, bradilian, sorbinicate, hexanicite, ronitol, and esters of nicotinic acid such as lower alcohol esters like methyl, ethyl, propyl or butyl esters. Each an any such derivative or compound will be collectively referred to hereinabove by ''nicotinic acid compound."

The specific HMG-CoA reductase inhibitors include, lovastatin and related compounds as disclosed in U.S. Patent No. 4,231.938, pravastatin and related compounds as reported in U.S. Patent Nos. 4,346,227 and 4,448,979, mevastatin and related compounds as disclosed in U.S. Patent No. 3,983,140, velostatin and simvastatin and related compounds as discussed in U.S. Patent Nos. 4,448,784 and 4,450,171, fluvastatin, atorvastatin, rivastatin and fluindostatin (Sandoz XU-62-320), with fluvastatin, lovastatin, pravastatin, atorvastatin, simvastatin and cerivastatin being preferred. Other HMG-CoA reductive inhibitors which may be employed herein include, pyrazole analogs of mevalonolactone derivatives as disclosed in U.S. Patent No. 4,613,610, indent analogs of mevalonolactone derivatives as disclosed in PCT application WO 86/03488, 6-[2-(substituted-pyrrol-1-yl)alkyl]pyran-2-ones and derivatives thereof as disclosed in U.S. Patent No. 4,647,576, Searle's SC-45355 (a 3-substituted pentanedioic acid derivative) dichloracetate, imidazole analogs of mevalonolactone as disclosed in PCT application WO 86/07054, 3-carboxy-2-hydroxy-propane-phosphoric acid derivatives as disclosed in French Patent No. 2,596,393, 2,3-di-substituted pyrrole, furan and thiophene derivatives as disclosed in European Patent Application No. 0221025 A 14, naphthyl analogs of mevalonolactone as disclosed in U.S. Patent No. 4,686,237, octahydro-naphthelenes such as disclosed in U.S. Patent No. 4,499,289, keto analogs of lovastatin as disclosed in European Patent Application No. 0142146 A2, as well as other known HMG-CoA reductase inhibitors, such as those disclosed in GB Patent Nos. 2,205,837 and 2,205,838; and in U.S. Patent Nos. 5,217,992; 5,196,440; 5,189,180; 5,166,364; 5,157,134; 5,110,940; 5,106,992; 5,099,035; 5,081,136; 5,049,696; 5,049,577; 5,025,017; 5,011,947; 5,010,105; 4,970,221; 4,940,800; 4,866,058; 4,686,237.

As stated hereinabove, HMG-CoA reductase inhibitors and nicotinic acid have been employed in the past for the treatment of hyperlipidemia, which condition is characterized by the presence of excess fats such as cholesterol and triglycerides, in the blood stream. According to the aspect of the present invention, an extended or sustained release composition of nicotinic acid coated with an immediate release coating of an HMG-CoA reductase inhibitor is prepared as an example. By "extended release" or "sustained release" it is understood to mean a composition which when orally administered to a patient to be treated, the active ingredient like an HMG-CoA reductase inhibitor, nicotinic acid, a nicotinic acid compound or mixtures thereof will be released for absorption into the blood stream over a period of time. For example, it is preferred that in a dosage of about 1500 milligrams (hereinafter "mgs") of nicotinic acid, approximately 100 percent of the nicotinic acid will be released to the blood stream in about 4 to about 8 hours and preferably within about 6 hours following ingestion.

While the nicotinic acid is released from the pharmaceutical combination in a sustained release manner, the HMG-CoA reductase inhibitors are formulated for immediate release following ingestion. By "immediate release," it is understood to mean that the HMG-CoA reductase inhibitor, which when orally administered to a patient to be treated, will be completely released from the composition for absorption into the blood stream within about 30 minutes following ingestion.

A specific sustained release composition according to the present invention employs an effective lipid-altering amount of nicotinic acid coated with an effective lipid-altering amount of an HMG-CoA reductase inhibitor. By "effective lipid-altering amount" or "effective antihyperlipidemic amount" it is understood to mean an amount which when orally administered to a patient to be treated, will have a beneficial effect upon the physiology of the patient, to include at least some lowering of, one or more of the following, total cholesterol. LDL-cholesterol, triglycerides and Lp(a) and at least some increase in HDL-cholesterol, and more particularly an increase in, e.g.. $HDL_2$-cholesterol and/or $HDL_3$-cholesterol, in the patient's blood stream. The beneficial effect will also include some decreases in the total cholesterol to HDL-cholesterol ratio and in the LDL-cholesterol-HDL-cholesterol ratio in the patient's blood stream. In some individuals, the beneficial effect may also include reduction in apolipoprotein B, reduction in apolipoprotein E and/or an increase in apolipoprotein A-I. An exemplary effective lipid-altering amount of nicotinic acid would be from about 250mg to about 3000mg of nicotinic acid to be administered according to the present invention, as will be more fully describe hereinbelow. An exemplary effective lipid-altering amount of an HMG-CoA reductase inhibitor would be from about 0.1mg to about 80 mg. These amounts will of course vary, dependent upon a number of variables, including the psychological needs of the patient to be treated.

Preferably, there is also included in a sustained release composition according to the present invention, a swelling or sustained release agent which is compounded with the nicotinic acid, and/or nicotinic acid compounds, such that when the composition is orally administered to the patient, the swelling agent will swell over time in the patient's gastrointestinal tract, and release the active nicotinic acid, and/or nicotinic acid compound over a period of time. As is known in the art, such swelling agents and amounts thereof, may be preselected in order to control the time release of the active nicotinic acid ingredient. Such swelling agents include, polymers such as sodium carboxymethylcellulose and ethylcellulose and waxes such as bees wax and natural materials such as gums and gelatins or mixtures of any of the above. Because the amount of the swelling agent will vary depending upon the nature of the agent, the time release needs of the patient and the like it is preferred to employ amounts of the agent which will accomplish the objects of the invention.

An exemplary and preferred swelling agent is hydroxy propyl methyl cellulose, in an amount ranging from about 5% to about 50% parts by weight per 100 parts by weight of tablet or formulation. A preferred example will ensure a sustained time release over a period of approximately 4-8 hours.

A binder may also be employed in the present compositions. While any known binding material is useful in the present invention, it is preferred to employ a material such as one or more of a group of polymers having the repeating unit of 1-ethenyl-2-pyrrolidinone. These polyvinyl pyrrolidinone polymers generally have molecular weights of between about 10.000 and 700,000, and are also known as "povidone or PVP."

Amounts of the binder material will of course, vary depending upon the nature of the binder and the amount of other ingredients of the composition. An exemplary amount of povidone in the present compositions would be from about 1 % to about 5% by weight of povidone per 100 parts by weight of the total formulation.

Processing aids such as lubricants, including stearic acid, magnesium stearate, glyceryl behenate, talc and colloidal silicon dioxide, may also be employed, as is known in the art. An exemplary amount of a lubricant, such as stearic acid, in the present compositions would be from about 0.5% to about 2.0% by weight per 100 parts by weight of tablet or formulation.

Also in accordance with the present invention, the sustained release compositions containing the nicotinic acid and/or nicotinic acid compounds are coated with an HMG-CoA reductase inhibitor for immediate release following oral administration. An exemplary coating in accordance with the present invention comprises an HMG-CoA reductase inhibitor, a plasticizer, film forming and/or coating agent and a coloring agent. Specific examples of plasticizers include, benzyl benzoate, chlorobutanol, dibutyl sebacate, diethyl phthalate, glycerin, mineral oil and lanolin alcohols, petrolatum and lanolin alcohols, polyethylene glycol, propylene glycol, sorbitol, triacetin and triethyl citrate. An exemplary amount of a plasticizer utilized in the coatings of the present invention would be from about 0.01 % to about 5% by weight of the tablet.

Specific examples of film forming and/or coating agents include, carboxymethylcellulose sodium, carnauba wax, cellulose acetate phthalate, cetyl alcohol, confectioner's sugar, ethylcellulose, gelatin, hydroxyethyl cellulose, hydroxy propyl cellulose, hydroxy propyl methyl cellulose, liquid glucose, maltodextrin, methyl cellulose, microcrystalline wax, polymethacrylates, polyvinyl alcohol, shellac, sucrose, talc, titanium dioxide and zein. An exemplary amount of a film forming/coating agent in the present coatings would be from about 0.01% to about 5% by weight of the tablet. Generally speaking to prepare a coating in accordance with the present invention, an HMG-CoA reductase inhibitor is suspended or dissolved in an aqueous-solution of polyethlene glycol and hydroxy propyl methyl cellulose and then sprayed on the sustained release tablets by a film-coating process to a thickness containing an effective antihyperlipidemic amount of an HMG-CoA reductase inhibitor. Examples of suitable coating thicknesses in accordance with the present invention are from about 0.1 mm to about 2.0mm or more.

Coated sustained release tablets of various sizes can be prepared, e.g., of about 265mg to 1650mg in total weight, containing both of the active substances in the ranges described above, with the remainder being a physiologically acceptable carrier of other materials according to accepted pharmaceutical practice. These coated tablets can, of course, be scored to provide for fractional doses. Gelatin capsules can be similarly formulated.

Consistent with the present invention, such dosage forms should be administered to individuals on a regimen of one dose per day, preferably during the evening hours.

Combinations of an HMG-CoA reductase inhibitor and nicotinic acid and/or nicotinic acid compounds in the same pharmaceutical are more convenient especially in the coated tablet form for oral administration.

Optionally, the oral pharmaceutical combinations of the present invention may include other active ingredients. In addition, the present invention contemplates that other active ingredients may be administered concurrently with the pharmaceutical combinations of the present invention. Examples of other active ingredients include anti-lipidemic agents and flush-inhibiting agents. Specific examples of anti-lipidemic agents include bile acid sequestrants, e.g., cholestyramine, colestipol DEAESephadex (Secholex® and Polidexide®), probucol and related compounds as disclosed in U.S. Patent No. 3,674,836, lipostabil (Rhone-Poulanc). Eisai E5050 (an N-substituted ethanolamine derivative), imanixil (HOE-402) tetrahydrolipstatin (THL), isitigmastanyl phosphorylcholine (SPC, Roche), aminocyclodextrin (Tanabe Seiyoku), Ajinomoto AJ-814 (azulene derivative), melinamide (Sumitomo). Sandoz 58-035, American Cyanimid CL-277.082 and CL-283,546 (disubstitutedurea derivatives), neomycin, p-aminosalicylic acid, aspirin, quarternary amine poly(diallyldimethylammonium chloride) and ionenes such as disclosed in U.S. Patent No. 4,027,009, poly(diallylmethylamine) derivatives such as disclosed in U.S. Patent No. 4,759,923, omega-3-fatty acids found in various fish oil supplements, fibric acid derivatives, e.g., gemfibrozil, clofibrate, bezafibrate, fenofibrate, ciprofibrate and clinofibrate, and other known serum cholesterol lowering agents such as those described in U.S. Patent No. 5,200,424; European Patent Application No. 0065835A1, European Patent No. 164-698-A, G.B. Patent No. 1.586.152 and G.B. Patent Application No. 2162-179-A.

Specific examples of flush-inhibitingagents include nonsteroidal anti-inflammatory drugs such as aspirin and salicylate salts: propionic acids such as ibuprofen, flurbiprofen, fenoprofen, ketoprofen, naproxen, sodium naproxen, carprofen and suprofen; indoleacetic acid derivatives such as indomethacin, etodolac and sulindac, benzeneacetic acids such as aclofenac, diclofenac and fenclofenac; pyrroleacetic acids such as zomepirac and tolmectin; pyrazoles such as phenylbutazone and oxyphenbutazone;oxicams such as piroxicam; and anthranilic acids such as meclofenamate and mefenamic acid.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

Additional illustrations of adjuvants which may be incorporated in the tablets are the following: a binder such as gum tragacanth, acacia, corn starch, potato starch, alginic acid or the like; a sweetening agent such as sucrose, aspartase, lactose or saccharin; a flavoring such as orange, peppermint, oil of wintergreen or cherry. Various other materials may be present as coatings or to otherwise modify the physical form of the dosage unit. For instance, tablets may be coated with shellac, sugar or both.

Some of the active agents described above form commonly known pharmaceutically acceptable salts, such as alkali metal and other common basic salts or acid addition salts, etc. References to the base agents are therefore intended to include those common salts known to be substantially equivalent to the parent compound.

In carrying out the objective of the present invention, the nicotinic acid, nicotinic acid compounds may be formulated into sustained release granules, sustained release particles, sustained release coated particles or sustained release beads or pellets according to any method known to the art for the manufacture of pharmaceutical compositions for incorporation into a variety of oral dosage forms suitable for oral use, such as tablets, such as rapidly disintegrating tablets, compression coated tablets, enteric coated tablets, capsules, caplets and sachets for sprinkle administration. In addition, the HMG-CoA inhibitors may be formulated into immediate release granules or immediate release coated raw materials for incorporation into the oral dosage forms of the present invention.

A preferred nicotinic acid sustained release dosage form is the Niaspan® tablets. The Niaspan® tablets may be coated with a coating containing an HMG-CoA reductase inhibitor in immediate release form to formulate a pharmaceutical combination of the present invention in which the nicotinic acid is in an extended release form and the HMG-CoA reductase inhibitor is in an immediate release form.

In a further embodiment, the pharmaceutical combination of the present invention may be enterically coated to delay disintegration and absorption in the gastrointestinal tract. For example. (1) sustained release nicotinic acid granules or immediate HMG-CoA reductase inhibitor granules may be individually enterically coated and compressed to form a tablet or a layer of a bilayer tablet, or (2) the tablet itself or a layer thereof may be coated with an enteric coating.

Enterically coated dosage forms do not necessarily dissolve or become absorbed by humans until they pass through the low pH environment of the stomach and pass into the relatively higher pH of the small intestine. Typical materials conventionally used as enteric coatings include, but are not limited to, cellulose acetate phthalate, polyvinylacetatephthalate, hydroxypropylmethylcellulose phthalate and methacrylic acid-methyl methacrylate copolymers. Such materials can be used individually or in combination. Additional formulating agents, such as plasticizers (e.g., one or more polyethylene glycols or propylene glycol) may be added to ensure physical strength and processability, e.g.. to prevent cracking due to stress, low humidity or other factors.

Enterically coated nicotinic acid or HMG-CoA reductase inhibitors granules can be prepared in a fluid bed granulator by coating or agglomeratingniacin powder with one or more enteric coating materials, such that microspheres or small particles of enterically coated nicotinic acid are formed. Alternatively, a whole tablet or capsule comprising an HMG-CoA reductase inhibitor and/or nicotinic acid can be coated with enteric coating materials.

Typically, the enteric coating process comprises coating the dosage form with a plurality of layers, e.g., one or two layers or more, of enteric coating material, like a methacrylate polymer such as EUDRAGIT S-100, available from Rohm, preferably by dipping the weight tablet or capsule into a freshly prepared solution of the material for five seconds. The solution of enteric coating material(s) may be prepared by dissolving an appropriate amount of material in, e.g., 100 ml of a 4:6 mixture of acetone and isopropyl alcohol. After each immersion, the coating is allowed to dry in air. e.g., for 30 minutes, prior to the next five-second immersion. A single coating is usually adequate to prevent the capsule or table from dissolving in the stomach. Alternatively, the granules, tablets or capsules may be coated or spray-dried in standard coating machines such as those typically employed in the pharmaceutical industry.

The present invention also contemplates methods for pretreating subjects, prior to the start of nicotinic acid or nicotinic acid combination therapy, with a nonsteroidal anti-inflammatory drug (NSAID) in an amount effective to inhibit or reduce prostaglandin $PGD_2$ synthesis, so that any flush reaction induced by the nicotinic acid therapy is lessened or prevented. In carrying out this aspect of the present invention, the pretreatment should start at least about 7 days prior to administration of the nicotinic acid, and preferably for at least about 14 days. While pretreatment for a shorter duration may not provide a subject with adequate protection against flushing, some protective effect may be observed and, thus, such shorter pretreatment periods may be practiced within the scope of the present invention.

During pretreatment of subjects with an NSAID, the NSAID selected is orally administered in at least one to four or more doses daily. However, while three or fewer doses per day is preferred, one or two doses per day are preferential for the convenience and improved compliance of the subjects. The NSAID may be administered orally as an immediate or extended release dosage form. Of course, if an extended release dosage form is selected, the NSAID can be administered fewer times daily then a comparable immediate release dosage form, while providing similar protection against nicotinic acid-induced flushing.

While it is preferable to take an NSAID during pretreatment, the present invention also contemplates continued administration of the NSAID during the nicotinic acid or nicotinic acid compound treatment. This can be accomplished by taking the NSAID as a separate dosage form on a daily basis, or by taking a pharmaceutical component of the present invention which includes an NSAID.

Particularly preferred NSAIDs include indomethacin, ibuprofen, naproxen, aspirin, ketoprofen, flurbiprofen, phenylbutazone, and piroxicam. These NSAIDs may be administered in their usual doses for treatment of inflammation. Aspirin is especially preferred. Aspirin may be administered in daily dosages of at least between about 60mg and about 1000mg, and more preferably at least between about 80mg and 650mg, and most preferably between about 80mg and 325mg. Even though higher daily dosages of aspirin may be consumed to suppress flushing in accordance with the present invention, there is risk that these higher dosages, as well as the high end of the preferred dosages, could induce gastrointestinal upset and ulceration.

While extended release forms are commercially available for some NSAIDs, other extended release formulations may be prepared by conventional methods from those versed in the art, or by blending the NSAID with the nicotinic acid during granules or during the powder blending stage pursuant to the methods described herein to generate a pharmaceutical combination comprised of nicotinic acid and an NSAID in extended release form. Alternatively, the NSAID could be blended with an HMG-CoA reductase inhibitor in a coating for immediate release of the NSAID. As a further alternative contemplated by the present invention, extended release nicotinic acid tablets, such as Niaspan®, can be enterically coated for delayed release, which then may be coated with a coat comprised of an HMG-CoA reductase and an NSAID for immediate release.

In a further aspect of the present invention, the solid pharmaceutical combinations for oral administration may be formulated into various shapes. For example, tablets may be round/flat, round/convex, oval/flat, oval/convex, or capsule (caplet) in shape, whereas capsules may be round or elongated in shape. It is presently believed that when tablets are coated in accordance with the present invention, the coatings can be improved if the tablets are in an oval/convex shape. For instance, it is believed that by formulating the sustained release nicotinic acid tablets, such as Niaspan® tablets, into oval/convex shapes, the coatings containing an HMG-CoA reductase inhibitor are improved, as compared to similar coatings on tablets having, for example, a capsule (caplet) shape.

The formulations as described above will be administered for a prolonged period, that is, for as long as the potential for elevated serum cholesterol and atherosclerosis remains or the symptoms continue. A dosing period of at least about 4 weeks maybe required to achieve a desired therapeutic benefit.

Examples of various embodiments of the present invention will now be further illustrated with reference to the following examples.

## Example I (reference)

In order to demonstrate the effectiveness of the compositions of the present invention over known antihyperlipidemia compositions and methods heretofore known in the art, a number of substantially identical composition were prepared according to the disclosure hereinabove. The composition ingredients and amounts are listed in TABLE IA hereinbelow.

**TABLE IA**
**Test Tablet Composition**

| Ingredient | 375mg | 500mg | 750mg |
|---|---|---|---|
| Nicotinic Acid | 375.0 | 500.0 | 750.0 |
| Hydroxy propyl methyl cellulose | 188.7 | 203.0 | 204.7 |
| Povidone | 12.9 | 17.2 | 25.9 |
| Stearic Acid | 5.8 | 7.3 | 9.9 |
| TOTAL | 582.4mg | 727.5mg | 990.5mg |

The ingredients were compounded together to form a tablet. More specifically. Niaspan® once-daily tablets in accordance with the present invention utilize a hydrophilic matrix controlled drug delivery system. This is a dynamic system composed of polymer wetting, polymer hydration and polymer disintegration/dissolution. The mechanism by which drug release is controlled depends on, for example, initial polymer wetting, expansion of the gel layer, tablet erosion and niacin solubility. After initial wetting, the hydrophilic polymer starts to partially hydrate, forming a gel layer. As water permeates into the tablet increasing the thickness of the gel layer, drug diffuses out of the gel layer. As the outer layer of the tablet becomes fully hydrated it erodes. It is believed that this erosion results in additional drug release. The controlled release from this matrix delivery system can be modified depending on the type and molecular weight of hydrophilic polymer used.

A Niaspan® formulation consists of Niacin. Methocel® E10M Premium, Povidone K90 and Hystrene 5016 (stearic acid). Methocel® E10M Premium is utilized as a controlled-release agent in the Niaspan® formulation. Methocel is a partly O-methylated and O-(2-hydroxypropylated) cellulose and is available in several grades which vary in terms of viscosity and degree of substitution. Methocel is manufactured by Dow Chemical.

Povidone K90 is employed as a granulating/binding agent in a Niaspan® formulation. Povidone is a synthetic polymer consisting of linear 1-vinyl-2-pyrrolidone groups, the degree of polymerization of which results in polymers of various molecular weights, or as indicated above. It is characterized by its viscosity in aqueous solution, relative to that of water, expressed as a K-value, ranging from 10-120. Povidone K90 has an approximate molecular weight of 1,000,000. Povidone is a hygroscopic, water soluble material. Povidone K90 present in a Niaspan® formulation is manufactured by ISP (International Speciality Products). Hystene 5016 is utilized as an external lubricant in the Niaspan® formulation. Hystrene 5016 is a mixture of stearic acid and palmitric acid. The content of stearic acid is not less than about 40.0% and the sum of the two acids is not less than about 90.0%. Hystrene 5016 is manufactured by Witco. Refer to Table IB for Niaspan® formulation details.

Qualitatively, the four tablet strength formulations are identical. The major component of each formulation is a granulated mixture of Niacin. Methocel E10M and Povidone K90. The granulation process improves compression properties.

**TABLE IB**

| Niaspan® Tablet Formulations | | | | |
|---|---|---|---|---|
| Niaspan® Product | 375mg Tablets | 500mg Tablets | 750m Tablets | 1000mg Tablets |
| Formulation, % Tablet | | | | |
| Niacin | 64.4 | 70.5 | 77.4 | 83.1 |
| Methocel E10M Premium (Intragranular) | 7.4 | 8.1 | 8.9 | 9.5 |
| Povidone K90 | 2.2 | 2.4 | 2.7 | 2.9 |
| Methocel E10M Premium (Extragranular) | 25.0 | 18.0 | 10.0 | 3.5 |
| Hystrene 5016 (Stearic Acid) | 1.0 | 1.0 | 1.0 | 1.0 |
| Tablet weight, mg | 582.5 | 709.5 | 968.6 | 1203.6 |

Niaspan® formulations are presented in white caplet shape tablets. Caplet dimensions differ with respect to product strength. The 375mg and 500mg Niaspan® tablets are compressed with tooling measuring approximately 0.687" in length x. 0.281 "by width. The length and width of the 750mg and 1000mg tooling measures approximately 0.750" x. 0.320". Target tablet weight and hardness dictate thickness across the four Niaspan® products. The production of the Niaspan® tablets will now be described generally as set forth below.

**Niaspan® Granulation Process Flow Chart**

| Raw Materials | Process Flow | Equipment |
|---|---|---|
| Niacin Povidone K90 Methocel E10M (Intragranular) Purified Water | Granulate | High shear granulator (Littleford FM130) |
| | l | |
| | Dry | Fluid bed drier (Glatt fluid bed drier) |
| | l | |
| | Parcel size reduction | Mill (Kemutec Betagrind) |

**Niaspan® Granulation Process Description**

Niaspan® granulation raw materials are dispensed and granulated in a high shear granulator. The wet granules are sieved into a fluid bed drier and are dried. When the drying process is complete, the granules are milled. Milling ensures uniform particle size distribution throughout the Niaspan® granulation.

**Niaspan® Tablet Process Flow Chart**

| Raw Materials | Process Flow | Equipment |
|---|---|---|
| | Niaspan® Tablet Blend | |
| Methocel E 10M (Extragranular) Hystrene 5016 (Stearic Acid) | Blend Milled Niaspan® granules with extragranular Methocel E 10M and Hystrene 5016 | Blender (Patterson-Kelley V-Blender) |
| | l | |
| | Niaspan® Tablet Manufacture | |
| | Compress Niaspan® Tablet Blend | Rotary tablet press |

**Niaspan® Tablet Process Description**

A Niaspan® tablet blend is manufactured by blending the Niaspan® granulation, extragranular Methocel E10M and Hystrene 5016. The quantities of each Niaspan® tablet blend component will depend on the particular Niaspan® dose being manufactured (refer to Table IB). A Niaspan® tablet blend is compressed to form Niaspan® tablets. Niaspan® tablet physical properties will vary depending on the particular Niaspan® dose being manufactured.

Production of Niaspan® tablets will now be discussed in greater detail. The initial stage of manufacturing is the same for all four tablet strengths of Niaspan® (375, 500, 750, and 100mg). One batch of Niaspan® granulation is comprised of four individual 40.0kg units of granulation which are processed separately, but under like conditions. The four individual granulations are sampled and tested individually and subsequently released for blending. The base granulation is not strength specific and may be used to manufacture any tablet strength of Niaspan®.

The ingredients in the base granulation are set forth in Table IC below:

**TABLE IC**

| Component | Function | Quantity per kilogram granulation (kg) | % per kilogram granulation | Quantity per 160.00 kg batch (kg) |
|---|---|---|---|---|
| Niacin, USP | Drug Substance | 0.87 | 87.00 | 139.20 |
| Povidone, USP | Binder | 0.03 | 3.00 | 4.80 |

(continued)

| Component | Function | Quantity per kilogram granulation (kg) | % per kilogram granulation | Quantity per 160.00 kg batch (kg) |
|---|---|---|---|---|
| Methocel USP, E10M Premium CR Grade | Controlled-Release Agent | 0.10 | 10.00 | 16.00 |
| Purified Water, USP* | Granulation Reagent | 0.00* | 0.00* | 48.00 |
| Total | | | | 160.00 |
| *Purified Water, USP is used as a granulation reagent and does not appear in the finished granulation. | | | | |

Raw materials are quantatively dispensed into appropriately labeled double polyethylene-lined containers using calibrated scales. Purified Water, USP is dispensed into an appropriate vessel from which it is later pumped during the wet-massing operation.

A Littleford FM130 granulator is charged with approximately one half of the Niacin, USP required for the process unit (~17.4kg) followed by about 4.00kg of Methocel, USP E 1 0M Premium CR Grade; about 1.20kg of Povidone, USP; and the balance of the Niacin. SP (~17.40kg). The powder bed is dry mixed in the Littleford FM130 granulator, with choppers on, for approximately I minute. At the completion of the 1-minute pre-mix cycle, about $12.0 \pm 0.05$kg of Purified Water, USP are sprayed onto the powder bed at a rate of about $2.40 \pm 0.24$kg/minute. Immediately following the addition of the Purified Water. USP, the unit is granulated for about 5 minutes.

The granulated unit is discharged into double polyethylene-lined containers and then manually loaded into a Glatt bowl while being passed through a #4 mesh screen. The Glatt bowl is loaded into a Glatt TFO-60 fluid-bed drier with an inlet air temperature setting of about $70°C \pm 5$ °C. The unit is dried until a moisture level of ≤ 1.0% is obtained as determined using a Computrac® Moisture Analyzer, model MA5A. The dried granulation is discharged inot appropriately labeled, double polyethylene-lined drums and reconciled.

The dried and reconciled granulation is passed through a Kemutec BetaGrind mill equipped with a 1.5mm screen and running at approximately 1500 RPM. The milled granulation is collected into appropriately labeled, double polyethylene-lineddrums and reconciled. The milled granulation is sampled and tested by Quality Control and released prior to further processing.

The released granulation units are charged to a Patterson-Kelley 20 ft$^3$ V-blender after which they are blended together for about $10 \pm 1$ minutes and then discharged to appropriately labeled, double polyethylene-lined containers.

As stated above, Niaspan® tablets are formulated from a common granulation which is blended with appropriate quantities of Methocel. USP E10M Premium CR Grade and Stearic Acid, NF to achieve the final dosage formulation. Tables IA and IB describe the formulation for each Niaspan® tablet strength, 375mg, 500mg, 750mg, and 1000mg, respectively.

Two study groups consisting of eleven and fourteen patients each were formed. Blood samples were taken from the patients, and tested for total cholesterol. LDL cholesterol, triglycerides and HDL cholesterol to establish baseline levels from which fluctuations in these lipids could be compared. The patients were then placed upon a regimen of the above discussed tables, totaling approximately 1500mg of nicotinic acid, once per day before going to bed. After eight weeks of this regimen, the patients were again tested for lipid profiles. The results of the tests conducted at eight weeks, showing the changes in the lipid profiles as a percentage change from the baseline, are reported in the table hereinbelow. Positive numbers reflect percentage increases and negative numbers reflect percentage decreases in this table.

**TABLE II**

| Patient Study Lipid Profile Data | | | | | | | |
|---|---|---|---|---|---|---|---|
| Pt. No. | Total-C | LDL-C | Apo B | Trigs | HDL-C | HDL$_2$-C | Lp(a) |
| Group A | | | | | | | |
| 1 | -8.2 | -12.0 | NA | -17.3 | 22.0 | NA | NA |
| 2 | -5.9 | -27.0 | NA | -28.7 | 65.0 | NA | NA |
| 3 | -15.1 | -13.0 | NA | -22.0 | -9.1 | NA | NA |
| 4 | -3.3 | -10.0 | NA | 61.6 | 3.8 | NA | NA |

(continued)

| Patient Study Lipid Profile Data | | | | | | | |
|---|---|---|---|---|---|---|---|
| Pt. No. | Total-C | LDL-C | Apo B | Trigs | HDL-C | $HDL_2$-C | Lp(a) |
| **Group A** | | | | | | | |
| 5 | -16.5 | -17.7 | NA | -28.8 | 11.1 | NA | NA |
| 6 | -12.4 | -25.9 | NA | -42.0 | 51.6 | NA | NA |
| 7 | -24.2 | -31.4 | NA | -39.4 | 12.5 | NA | NA |
| 8 | -6.7 | -7.4 | NA | -42.4 | 18.8 | NA | NA |
| 9 | 4.5 | 1.1 | NA | 7.2 | 9.2 | NA | NA |
| 10 | 2.8 | -0.2 | NA | -2.7 | 22.9 | NA | NA |
| 11 | -13.0 | -9.4 | NA | -54.0 | 44.3 | NA | NA |
| Mean | -8.9 | -13.9 | NA | -18.9 | 23.0 | NA | NA |
| p-Value | 0.0004 | 0.0001 | | 0.0371 | 0.0068 | | |
| **Group B** | | | | | | | |
| 1 | -19.2 | -27.1 | -24.4 | -33.4 | 20.0 | 22.3 | -81.9 |
| 2 | -32.2 | -35.7 | -28.0 | -60.4 | 4.3 | 3.2 | -25.3 |
| 3 | -21.4 | -33.6 | -35.6 | -33.4 | 30.4 | 38.6 | -17.4 |
| 4 | -19.9 | -24.6 | -15.1 | -20.8 | 9.6 | 16.1 | -27.0 |
| 5 | -3.3 | -2.1 | -29.4 | -41.1 | 5.8 | 2.4 | -22.4 |
| 6 | | | Patient Withdrew From Study | | | | |
| 7 | 23.1 | -32.6 | -42.6 | -58.6 | 49.2 | 68.9 | -14.3 |
| 8 | 24.8 | 34.0 | -28.4 | 5.5 | 6.5 | -6.8 | NA |
| 9 | 10.1 | 12.0 | -16.8 | -11.6 | 20.7 | -12.3 | 40.6 |
| 10 | -2.9 | -7.7 | -28.0 | -59.0 | 53.1 | 70.5 | -41.2 |
| 11 | -10.5 | -18.8 | -25.3 | -53.4 | 31.8 | 39.7 | NA |
| 12 | -20.0 | -30.8 | -30.4 | 11.7 | 21.1 | 25.0 | -28.4 |
| 13 | 17.4 | 16.8 | -17.5 | -17.5 | 51.3 | 51.9 | 38.5 |
| 14 | -9.4 | -16.6 | -32.0 | -46.9 | 52.3 | 67.6 | 17.6 |
| Mean | -8.7 | -12.8 | -32.2 | -27.2 | 25.3 | 30.1 | -17.9 |
| p-Value | 0.0002 | <0.0001 | 0.0001 | <0.0001 | <0.0002 | 0.0002 | <0.0188 |
| Combined | -8.7 | -13.3 | Gp B | -26.1 | 25.3 | Gp B | Gp B |
| p-Value | 0.0002 | <0.0001 | only | <.0001 | <0.0001 | only | only |

The data reported in TABLE II shows that the LDL levels in the Group A patients had a mean decrease of -13.9% and triglyceride decrease of - 18.9% HDL cholesterol levels, the beneficial cholesterol, were raised by 23.0% in this Group. Similar results were obtained with the Group B patients. These studies demonstrate that dosing the sustained release formulation during the evening hours or at night provides reductions in LDL cholesterol levels equal to immediate release niacin on a milligram per milligram basis, but superior reductions in triglyceride reductions when compared to sustained release formulations dosed during daytime hours on a milligram per milligram basis. Additionally, the increases in HDL cholesterol obtained from dosing the sustained release formulation during the evening or a night were +23.0% for one group and +25.3% for the other group. Dosing during the evening therefore provides reduction in LDL cholesterol plus significant decreases in triglycerides and increases in HDL cholesterol with once-a-day dosing.

Groups A and B were also tested for liver enzymes (AST, ALT and Alkaline Phosphatase), uric acid and fasting glucose levels at the start of the study described hereinabove (to form a baseline) and at two, four and eight week intervals. The results of these tests are listed in TABLES III-VII hereinbelow.

**TABLE III**

| THE EFFECT OF NIASPAN® THERAPY ON AST (SGOT) LEVELS (U/L) (1500 mgs dosed once-a-day at night) (n = 28) Weeks of Therapy With NIASPAN® | | | | | |
|---|---|---|---|---|---|
| Pt # | Baseline | 2 Wks. | 4 Wks. | 8 Wks. | Reference Range |
| **Group A** | | | | | |
| 1 | 28 | 29 | 25 | 25 | 0-50 |
| 2 | 24 | 25 | 24 | 26 | 0-50 |
| 3 | 17 | 18 | 22 | 21 | 0-50 |
| 4 | 14 | 16 | 15 | 17 | 0-50 |
| 5 | 22 | NA | 32 | 52 | 0-50 |
| 6 | 21 | 17 | 17 | 14 | 0-50 |
| 7 | 17 | 17 | 14 | 18 | 0-50 |
| 8 | 20 | 21 | 22 | 22 | 0-50 |
| 9 | 16 | 16 | 17 | 20 | 0-50 |
| 10 | 18 | 21 | 21 | 25 | 0-50 |
| 11 | 21 | 21 | 22 | 21 | 0-50 |
| **Group B** | | | | | |
| 1 | 23 | 25 | 38 | 33 | 0-50 |
| 2 | 20 | 20 | 21 | 21 | 0-50 |
| 3 | 15 | 20 | 18 | 19 | 0-50 |
| 4 | 25 | 22 | 25 | 26 | 0-50 |
| 5 | 23 | 21 | 17 | 18 | 0-50 |
| 6 | PATIENT WITHDREW DUE TO FLUSHING | | | | |
| 7 | 21 | 18 | 18 | 19 | 0-50 |
| 8 | 18 | 19 | 18 | 19 | 0-50 |
| 9 | 15 | 16 | 18 | 15 | 0-50 |
| 10 | 16 | 15 | 19 | 28 | 0-50 |
| 11 | 20 | 22 | 24 | 28 | 0-50 |
| 12 | 23 | 25 | 28 | 22 | 0-50 |
| 13 | 20 | 15 | 20 | 19 | 0-50 |
| 14 | 18 | 25 | 20 | 18 | 0-50 |
| Combined Mean | 19.8 | 20.4 | 20.8 | 21.1 | |
| Change From Baseline | | +3.0% | +5.1% | +6.6% | |
| Level of Significance: p=0.4141 | | | | | |

**TABLE IV**

| THE EFFECT OF NIASPAN® THERAPY ON ALT (SGPT) LEVELS (U/L) (1500 mgs dosed once-a-day at night) (n = 28) Weeks Of Therapy With Niaspan® | | | | | |
|---|---|---|---|---|---|
| Pt # | Baseline | 2 Wks. | 4 Wks. | 8 Wks. | Reference Range |
| **Group A** | | | | | |
| 1 | 32 | 28 | 39 | 30 | 0-55 |
| 2 | 24 | 25 | 23 | 26 | 0-55 |
| 3 | 18 | 23 | 30 | 30 | 0-55 |
| 4 | 7 | 13 | 14 | 14 | 0-55 |
| 5 | 14 | NA | 43 | 46 | 0-55 |
| 6 | 12 | 11 | 14 | 10 | 0-55 |
| 7 | 9 | 7 | 11 | 7 | 0-55 |
| 8 | 16 | 18 | 23 | 21 | 0-55 |
| 9 | 14 | 17 | 20 | 14 | 0-55 |
| 10 | 14 | 15 | 17 | 19 | 0-55 |
| 11 | 18 | 18 | 20 | 16 | 0-55 |
| **Group B** | | | | | |
| 1 | 16 | 17 | 27 | 29 | 0-55 |
| 2 | 16 | 14 | 15 | 22 | 0-55 |
| 3 | 13 | 21 | 13 | 16 | 0-55 |
| 4 | 23 | 20 | 26 | 17 | 0-55 |
| 5 | 21 | 23 | 17 | 15 | 0-55 |
| 6 | PATIENT WITHDREW DUE TO FLUSHING | | | | |
| 7 | 21 | 16 | 18 | 21 | 0-55 |
| 8 | 18 | 20 | 17 | 18 | 0-55 |
| 9 | 11 | 5 | 11 | 8 | 0-55 |
| 10 | 8 | 10 | 14 | 17 | 0-55 |
| 11 | 17 | 12 | 18 | 16 | 0-55 |
| 12 | 14 | 18 | 20 | 16 | 0-55 |
| 13 | 14 | NA | 11 | 10 | 0-55 |
| 14 | 23 | 23 | 19 | 19 | 0-55 |
| Combined Mean | 17.7 | 17.5 | 19.3 | 18.2 | |
| Change From Baseline | | -1.1% | 9.0% | +2.8% | |
| Level of Significance: p=0.3424 | | | | | |

**TABLE V**

| THE EFFECT OF NIASPAN® THERAPY ON ALKALINE PHOSPHATE LEVELS (U/L) 0 mgs dosed once-a-day at night) (n = 28) Weeks Of Therapy With Niaspan® | | | | | |
|---|---|---|---|---|---|
| P.t. # | Baseline | 2 Wks. | 4 Wks. | 8 Wks. | Reference Range |
| **Group A** | | | | | |
| 1 | 52 | 56 | 57 | 55 | 20-140 |
| 2 | 103 | 100 | 89 | 102 | 20-140 |
| 3 | 54 | 45 | 53 | 51 | 20-140 |
| 4 | 70 | 68 | 71 | 91 | 20-140 |
| 5 | 77 | NA | 74 | 81 | 20-140 |
| 6 | 55 | 48 | 49 | 51 | 20-140 |
| 7 | 72 | 71 | 79 | 75 | 20-140 |
| 8 | 55 | 49 | 47 | 50 | 20-140 |
| 9 | 53 | 55 | 56 | 45 | 20-140 |
| 10 | 74 | 73 | 75 | 75 | 20-140 |
| 11 | 18 | 18 | 20 | 16 | 20-140 |
| **Group B** | | | | | |
| 1 | 73 | 67 | 89 | 95 | 20-140 |
| 2 | 82 | 64 | 72 | 71 | 20-140 |
| 3 | 73 | 69 | 72 | 82 | 20-140 |
| 4 | 37 | 36 | 37 | 38 | 20-140 |
| 5 | 65 | 53 | 54 | 61 | 20-140 |
| 6 | PATIENT WITHDREW DUE TO FLUSHING | | | | |
| 7 | 64 | 58 | 58 | 58 | 20-140 |
| 8 | 79 | 78 | 65 | 73 | 20-140 |
| 9 | 94 | 92 | 103 | 93 | 20-140 |
| 10 | 69 | 67 | 70 | 65 | 20-140 |
| 11 | 59 | 67 | 63 | 72 | 20-140 |
| 12 | 65 | 59 | 59 | 63 | 20-140 |
| 13 | 64 | 68 | 66 | 64 | 20-140 |
| 14 | 72 | 61 | 59 | 64 | 20-140 |
| Combined Mean | 66.5 | 61.5 | 63.3 | 65.8 | |
| Change From Baseline | | -6.1% | -3.4% | +0.005% | |
| Level of Significance: p=0.0236 | | | | | |

TABLE VI

| THE EFFECT OF NIASPAN® ON URIC ACID LEVELS (mg/dL) (1500 mgs dosed once-a-day at night) (n = 28) Weeks Of Therapy With NISPAN® | | | | | |
|---|---|---|---|---|---|
| Pt# | Baseline | 2 Wks. | 4 Wks. | 8 Wks. | Reference Range |
| **Group A** | | | | | |
| 1 | 5.2 | 5.0 | 4.8 | 4.3 | 4.0-8.5 |
| 2 | 4.0 | 4.6 | 4.5 | 6.2 | 2.5-7.5 |
| 3 | 6.3 | 7.0 | 6.5 | 6.2 | 4.0-8.5 |
| 4 | 3.1 | 4.6 | 4.2 | 3.8 | 2-5-7.5 |
| 5 | 3.4 | NA | 3.3 | 4.2 | 2.5-7.5 |
| 6 | 6.6 | 5.5 | 5.6 | 4.7 | 4.0-8.5 |
| 7 | 3.8 | 4.5 | 4.3 | 4.9 | 2.5-7.5 |
| 8 | 4.4 | 3.8 | 5.1 | 4.5 | 2.5-7.5 |
| 9 | 3.9 | 4.5 | 4.6 | 3.5 | 2.5-7.5 |
| 10 | 2.6 | 2.9 | 2.8 | 2.7 | 2.5-7.5 |
| 11 | 4.7 | 5.5 | 5.2 | 5.3 | 2.5-75 |
| **Group B** | | | | | |
| 1 | 3.7 | 4.2 | 4.7 | 3.5 | 2.5-7.5 |
| 2 | 2.8 | 3.5 | 3.6 | 2.3 | 4.0-8.5 |
| 3 | 4.2 | 5.3 | 5.5 | 5.3 | 2.5-7.5 |
| 4 | 4.7 | 3.9 | 5.1 | 3.6 | 4.0-8.5 |
| 5 | 3.7 | 4.1 | 4.1 | 3.8 | 2.5-7.5 |
| 6 | PATIENT WITHDREW DUE TO FLUSHING | | | | |
| 7 | 5.8 | 6.6 | 6.6 | 6.8 | 2.5-7.5 |
| 8 | 4.7 | 4.3 | 6.=1 | 5.6 | 2.5-7.5 |
| 9 | 3.7 | 4.6 | 5.1 | 3.8 | 2.5-7.5 |
| 10 | 4.2 | 5.0 | 4.4 | 8.5 | 2.5-7.5 |
| 11 | 1.9 | 3.0 | 2.8 | 5.0 | 2.5-7.5 |
| 12 | 5.6 | 5.4 | 6.2 | 5.6 | 4.0-8.5 |
| 13 | 4.2 | 4.6 | 4.6 | 5.3 | 2.5-7.5 |
| 14 | 5.5 | 5.4 | 6.1 | 5.3 | 2.5-7.5 |
| Combined Mean | 4.54 | 4.82 | 4.92 | 4.86 | *p=0.3450 |
| Change From Baseline | | +6.2% | +8.4% | +7.0% | |
| *Level of Significance: p = 0.3450 | | | | | |

**TABLE VII**

| THE EFFECT OF NIASPAN® THERAPY ON FASTING GLUCOSE LEVELS (mg/dL) (1500 mgs dosed once-a-day at night) (n = 28) Weeks Of Therapy With NIASPAN® | | | | | |
|---|---|---|---|---|---|
| Pt # | Baseline | 2 Wks. | 4 Wks. | 8 Wks. | Reference Range |
| Group A | | | | | |
| 1 | 114 | 122 | 123 | 110 | 70-115 |
| 2 | 101 | 105 | 107 | 101 | 80-125 |
| 3 | 99 | 98 | 109 | 103 | 70-115 |
| 4 | 100 | 118 | 94 | 94 | 80-125 |
| 5 | 89 | NA | 82 | 103 | 80-125 |
| 6 | 97 | 103 | 94 | 107 | 70-115 |
| 7 | 85 | 107 | 100 | 94 | 80-125 |
| 8 | 98 | 107 | 103 | 101 | 80-125 |
| 9 | 97 | 97 | 100 | 110 | 80-125 |
| 10 | 94 | 101 | 111 | 97 | 70-115 |
| 11 | 102 | 103 | 95 | 95 | 80-125 |
| Group B | | | | | |
| 1 | 101 | 97 | 83 | 99 | 70-115 |
| 2 | 90 | 95 | 96 | 89 | 80-125 |
| 3 | 96 | 98 | 95 | 97 | 70-125 |
| 4 | 116 | 139 | 113 | 125 | 80-125 |
| 5 | 88 | 92 | 91 | 95 | 70-115 |
| 6 | PATIENT WITHDREW DUE TO FLUSHING | | | | |
| 7 | 106 | 114 | 118 | 117 | 70-115 |
| 8 | 95 | 106 | 106 | 108 | 70-115 |
| 9 | 81 | 92 | 84 | 92 | 70-115 |
| 10 | 108 | 117 | 122 | 105 | 70-115 |
| 11 | 85 | 106 | 106 | 108 | 70-115 |
| 12 | 92 | 89 | 101 | 86 | 80-125 |
| 13 | 99 | 105 | 94 | 100 | 70-125 |
| 14 | 100 | 108 | 84 | 107 | 70-125 |
| Combined Mean | 98.4 | 105.8 | 101.6 | 102.3 | |
| Change From Baseline | | +7.5% | -3.3% | +4.0% | |
| Level of Significance: p = 0.0021 | | | | | |

In order to provide a comparison between the state of the art prior to the present invention, and in order to quantify the magnitude of the improvement that the invention provides over the prior art, another study was conducted. This study included 240 patients dosed according to the present invention as described hereinabove. Compared to this group was the group of patients studied by McKenney et al., as reported hereinabove. The results of this study are reported in TABLE VIII hereinbelow.

**TABLE VIII**

| A Comparison of Changes in Liver Function Tests | | | | | | | | |
|---|---|---|---|---|---|---|---|---|
| | **0** | **500** | **1000** | **1500** | **2000** | **2500** | **3000** | **TOTAL** |
| **McKenney SR[b] Niacin** | | | | | | | | |
| AST | 23.8 | 27.9 | 40.4 | 36.6 | 56.5 | na | 97.0 | |
| % | -- | 117 | 170 | 154 | 237 | na | 408 | |
| **Invention Dosage[c]** | | | | | | | | |
| AST | 24.3 | na | 23.7 | 27.5 | 26.6 | 27.6 | 27.8 | |
| % | -- | na | 98 | 113 | 109 | 114 | 114 | |
| **McKenney SR Niacin** | | | | | | | | |
| AST | 25.6 | 29.5 | 36.3 | 39.0 | 59.1 | na | 100.0 | |
| % | -- | 115 | 142 | 152 | 231 | na | 391 | |
| **Invention Dosage** | | | | | | | | |
| ALT | 21.4 | na | 18.7 | 22.6 | 21.3 | 22.4 | 21.8 | |
| % | -- | na | 87 | 106 | 100 | 105 | 102 | |
| **McKenney SR Niacin** | | | | | | | | |
| ALK | 95 | 95 | 106 | 105 | 136 | na | 135 | |
| % | -- | 100 | 112 | 111 | 143 | na | 142 | |
| **Invention Dosage** | | | | | | | | |
| ALK. | 74.7 | na | 73.9 | 76.1 | 73.4 | 76.7 | 78.0 | |
| % | -- | na | 99 | 102 | 98 | 103 | 104 | |
| **McKenney SR Niacin** | | | | | | | | |
| Drop | -- | 0 | 2 | 2 | 7 | na | 7 | 18 |
| n | -- | -- | -- | -- | -- | -- | -- | 23 |
| % | -- | 0 | 9 | 9 | 30 | na | 30 | 78 |
| **Invention Dosage** | | | | | | | | |
| Drop | -- | -- | 0 | 0 | 0 | 0 | 0 | 0 |
| n | -- | -- | 26 | 67 | 97 | 35 | 15 | 240 |
| % | -- | -- | 0 | 0 | 0 | 0 | 0 | 0 |
| 1 year | -- | -- | 15 | 47 | 77 | 31 | 15 | 184 |
| 1 year | -- | -- | 58 | 69 | 79 | 89 | 100 | 77 |
| Dosed twice-per-day as described in "A Comparison of the Efficacy and Toxic Effects of Sustained - vs. Immediate - Release Niacin in Hypercholesterolemic Patients" by McKenney et al. Journal of the American Medical Association, March 2, 1994: Vol. 271. No. 9, pages 672-677. [b] SR is 'sustained release" [c] Dosed once-per-day at night | | | | | | | | |

The results of the comparison of the studies reported in TABLE VIII show that the control group (the MeKenney group) had 18 of 23, or 78 percent of the patients therein drop out of the test because of an increase in their respective liver function tests. The patients withdrew at the direction of the investigator. In comparison, a group of 240 patients treated according to the present invention had zero patients drop out, based upon the same criteria for withdrawal. The tests results reported above indicate that this sustained release dosage form caused no elevation in liver function tests (i.e. no liver damage), no elevations in uric acid and only a small, 7.5% increase in fasting glucose levels which in fact decreased during continued therapy.

Thus, it should be evident that the compositions and method of the present invention are highly effective in controlling hyperlipidemia in hyperlipidemics, by reducing the levels of LDL cholesterol, triglyceride and Lp(a) while increasing HDL cholesterol levels. The present invention is also demonstrated not to cause elevations in liver function tests, uric acid or glucose levels for the hyperlipidemics.

## Example II

In order to demonstrate the effectiveness of the pharmaceutical combinations and methods of the present invention over an antihyperlipidemia compound and method, nicotinic acid sustained release compositions coated with different HMG-CoA reductase inhibitors are prepared according to the disclosure hereinabove and hereinbelow. The composition ingredients and amounts are listed in Table IXA and IXB and the results of the study are recited in Tables X and XI hereinbelow.

### TABLE IXA

| Coated Tablet Composition | | | |
|---|---|---|---|
| Ingredient | 500mg | 750mg | 1000mg |
| Core Tablet | --- | --- | ---- |
| Nicotinic Acid | 500 | 750 | 1000 |
| Hydroxypropyl methylcellulose (Methocel E10) | 203 | 183.1 | 157 |
| Povidone | 17.2 | 25.8 | 34.5 |
| Stearic Acid | 7.3 | 9.7 | 12.1 |
| Core Tablet Weight | 727.5mg | 990.5mg | 1203.6 |
| Lovastatin | 10.mg | 10mg | 10mg |
| Polyethylene Glycol | 0.9mg | 0.9mg | 0.9mg |
| Hydroxypropyl methylcellulose (Methocel E5) | 29.1mg | 29.1mg | 29.1mg |
| Coating Weight | 40mg | 40mg | 40mg |
| Total Tablet Weight | 767.5 | 1030.5 | 1243.6 |

### TABLE IXB

| Batch Formulation | | | | |
|---|---|---|---|---|
| | Niacin 750mg | | Niacin 1000mg | |
| | Lovastatin 10mg | | Lovastatin 10mg | |
| Material | Per Unit MG/Tablet | Per Batch, G | Per Unit MG/Tablet | Per Batch, G |
| Lovastatin | 10.0 | 80.54 | 10.0 | 64.74 |
| Methocel E5 Premium. LV | 29.1 | 234.35 | 19.4 | 125.60 |
| Pluracol E1450 | 0.9 | 7.25 | 0.6 | 3.88 |
| Purified Water | na | 2899.26 | na | 1942.20 |
| Coating Suspension Total | na | 3221.4 | na | 2136.42 |
| Niacin 750mg Core Tablet | 968.5 | 6000.0 | 1203.6 | 6000.0 |
| Total | 1008.5 | 9221.4 | 1233.6 | 8136.42 |

The core tablet ingredients are compounded together to form sustained release tablets, as described in Example I. The sustained release tablets are then coated as follows. The lovastatin. Methocel E5 and Pluracol E1450 are pre-blended in a polyethylene bag for about 2-3 minutes. The mixture is then passed through a 710mm sieve. A low sheer propeller blade mixer is positioned in a stainless steel beaker containing purified water, USP. The mixer speed is adjusted until a vortex forms. The blended mixture in the polyethylene bag is slowly added to the purified water. If necessary, the mixer speed should be adjusted during the addition of the dry mixture so that the vortex conditions are maintained. Continue mixing until the blended material is completely dispersed.

Place the stainless steel beaker on a balance and record gross weight. Calculate net weight of coating suspension as follows:

$$\text{Net weight of coating suspension} = \text{gross weight of coating suspension} - \text{beaker tare weight}$$

Following manufacture of the coating suspension, the sustained release tablets are coated as follows. The Hicoater HCT 48/60 tablet coating machine is first cleaned appropriately pursuant to SOP FM700-Procedure for the cleaning of Hicoater HCT 48/60 tablet coating machine. The Hicoater HCT 48/60 tablet coating machine should be equipped with a 9 liter pan, 0.6cc gear prop, single gun spray bar, 2.5mm cap and 1.5mm nozzle port.

Following SOP FM500 - Procedure for the operators of the Hicoater HCT 48/60 tablet coating machine in manual mode, the atomization air pressure should be set to 150 liter/min and the pattern air pressure should be set to 100 liters/min. Once the atomization air pressure and the pattern air pressure are set, the coating suspension is placed on a balance and the suspension feed line is placed in the coating suspension. The suspension return line is then placed in another container. The low sheer mixer is then placed in the coating suspension and the mixing is started. A period of about 60 minutes should be allowed before proceeding to the next step.

After about 60 minutes, the suspension pump and purge lines are switched on. When the lines are filled with coating suspension, relocate the suspension return line to the coating suspension container. The solution following through the guns should be set to about 40g/min according to SOP FM500.

Next, the batch of nicotinic acid sustained release tablets are loaded into the coating machine. Close the glass door on the machine. Start the inlet and exhaust air blowers. Adjust the inlet and exhaust air blower until air flow is 170 ($\pm 20$)cfm and pan pressure negative is between - ½ inch and 1 inch.

Coat the tablets as follows. Set the pan to JOG at 3.3rpm. 5 seconds on and 30 seconds off. Switch on the inlet air heater and adjust to 60°C. Proceed to the film coating phase where the exhaust air temperature reaches 40°C. To further coat, set the pan to run. Increase the pan speed to 15rpm and start the spray. Calculate the coating end point or target coated tablet weight as follows:

| Coating end point (750mg) | = | Starting tablet weight, mg x 1.0413 for 750 mg tablets |
|---|---|---|
| Coating end point (1000mg) | = | Starting tablet weight, mg x 1.0249 for 1000mg tablets |
| Coating end point (500mg) | = | Starting tablet weight, mg x 1.0643 for 500 mg tablets |

The coating end point should be approximately $\pm 10\%$ of the target coated tablet weight range.

Continue to apply coating suspension until the end point is reached. Proceed to the next step, which is cooling upon reaching the end point.

To cool, stop the spray. Set the pan to JOG at 3.3rpm. Switch off the inlet air heat and allow the coated tablets to cool to approximately 35°C. Stop the pan and turn off the inlet and exhaust blowers.

To discharge, use the JOG button on the front of the machine to turn the pan until the trap door is above the surface of the product bed. Position a tared double polyethylene lined container with desiccant present in the outer bag beneath the discharge chute. Open the trap door. Rotate the JOG button until coated tablets begin to discharge. Continue to rotate the pan until all the product is discharged from the pan. Stop the pan and remove the container. Then weigh the coated sustained release tablets.

**Example III** (Reference)

A study group consisting of 382 patients was formed. Blood samples were taken from the patients, and were tested for total cholesterol, LDL-cholesterol, triglycerides and HDL-cholesterol to establish baseline levels from which fluctuations in these lipids could be composed. The patients were then placed upon a regimen as follows: Of the 382 patients, 258 patients took approximately 2000mg of Niaspan®, once per day before going to bed, and 122 of 124 patients took concomitantly, once per day at night before going to bed, approximately 2000mg of Niaspan® (two Niaspan® 1000 mg tablets) and one HMG-CoA reductase inhibitor tablet, as reported in Table X. More specifically, 4 patients took two Niaspan® 1000mg tablets and one fluvastatin 20mg tablet at the same time once per day at bedtime; 12 patients took two Niaspan® 1000mg tablets one lovastatin 20mg tablet at the same time once per day at night before going to bed; 69 patients took two Niaspan® 1000mg tablets and one pravastatin 20mg tablet at the same time once per day at night before going to bed; 27 patients took two Niaspan® 1000mg tablets and one simvastatin 10mg tablet at the same time once per day at night before going to bed; and 10 patients took two Niaspan® 1000mg tablets and one HMG-CoA reductase tablet at the same time once per day at night before going to bed. However, during the study, these 10 patients changed between different HMG-CoA reductase inhibitors. Nevertheless, the particular HMG-CoA reductase inhibitors taken by these 10 patients were those recited in Table X.

After treatment, with a mean treatment duration of approximately 43 weeks, the lipid profiles of the patients were again tested. The results of the tests conducted, showing the change in the lipid profiles as percentage change from the baseline, are reported in Tables X and XI hereinbelow. The results of the tests conducted, showing the change in clinical chemistry profiles as percentage change from baseline, are reported in Table XII hereinbelow, and showing the number of patients and the % of the total patients in the study that recorded elevations above upper limits of normal (ULN) for selected clinical chemistry parameters, are reported in Tables XIII and XIV hereinbelow.

No incidences or symptoms of myopathy or rhabdomyolysis were described by or observed in the 122 individuals receiving the combination therapy pursuant to this Example III.

**TABLE X,**

| NIASPAN® AND NIASPAN®/HMG-CoA REDUCTASE INHIBITOR LONG-TERM STUDY EFFICACY DATA: MEAN TREATMENT DURATION - ABOUT 43 WEEKS | | | | | | | |
|---|---|---|---|---|---|---|---|
| | | % Change from Baseline | | | | Median Dose (mg) | |
| | N | TC | LDL-C | HDL-C | TG | Niaspan ® | Statin |
| Niaspan® | 258 | -12.4 | -19.1 | +26.0 | -25.5 | 2000 | -- |
| Niaspan® + Statin Total | 122 | -23.8 | -31.8 | +27.7 | -32.5 | 2000 | -- |
| Fluvastatin | 4 | -22.1 | -31.8 | +29.3 | -30.3 | 2000 | 20 |
| Lovastatin | 12 | -20.9 | -28.2 | +23.5 | -23.8 | 2000 | 20 |
| Pravastatin | 69 | -23.7 | -31.4 | +26.5 | -34.5 | 2000 | 20 |
| Simvastatin | 27 | -24.9 | -33.0 | +33.9 | -36.4 | 2000 | 10 |
| Multiple | 10 | -25.3 | -35.1 | +23.7 | -19.8 | 2000 | -- |

Table XI also reports results of the tests conducted. More specifically, Table XI reports complete efficacy data (lipid results) for 53 of the 124 patients, who took concomitantly, once per day at night before going to bed, Niaspan® and an HMG-CoA reductase inhibitor, as indicated above in this Example III. Table XI further reports complete efficacy data (lipid results) for 16 patient, who took concomitantly, once per day at night before going to bed. Niaspan® and BAS, a bile acid sequestrant (i.e., cholestyramine or colestipol). Table XI also reports complete efficacy data (lipid results) for 15 patients, who took concomitantly, once per day at night before going to bed, Niaspan®, BAS (a bile acid sequestrant, i.e., cholestyramine or colestipol), and an HMG-CoA reductase inhibitor.

**TABLE XI**

**LIPID RESULTS** Mean Percentage Change from Baseline LONG TERM POPULATION

| | | n | Subgroup Total | n | Niaspan® Only | n | Niaspan® + HmgCoA | n | Niaspan® +BAS | n | Niaspan® +Both |
|---|---|---|---|---|---|---|---|---|---|---|---|
| Total # of Patients | | | 269 | | 185 | | 53 | | 16 | | 15 |
| LDL | Baseline | 269 | 201.0±1.9 | 185 | 198.2±2/1 | 53 | 208.0±4.7 | 16 | 207.1±11.1 | 15 | 203.7±8.8 |
| (mg/dL) | 12 weeks | 234 | (-10.7±0.84)** | 150 | (-11.4±1.03)** | 53 | (-8.6±1.85)** | 16 | (-11.2±3.42)** | 15 | (-10.3±3.32)** |
| | 24 weeks | 208 | (-14.5±0.98)** | 126 | (-13.4±1.16)** | 52 | (-16.0±2.43)** | 16 | (-14.8±3.24)** | 14 | (-18.2±2.53)** |
| | 48 weeks | 174 | (-21.5±1.10)** | 101 | (-17.7±1.28)** | 45 | (-31.7±2.12)** | 15 | (-19.5±3..73)** | 13 | (-18.6±3.50)** |
| | 72 weeks | 140 | (-23.2+ 1.19)** | 79 | (-18.1±1.52)** | 39 | (-31.6±1.73)** | 9 | (-30.9±3.83)** | 13 | (-24.0±4.12)** |
| | 96 weeks | 130 | (-23.8+1.37)** | 73 | (-17.5+1.65)** | 37 | (]2.2+2.32)** | 7 | (-27.8±4.19)** | 13 | (-33.0+4.18>** |
| IIDL | Base4ine | 269 | 434±0.6 | 185 | -43-6±0.7 | 53 | 42.7±1.4 | 16 | 42.5±2.3 | 15 | 44.0±2.2 |
| (mg/dL) | 12 weeks | 234 | (19.6±1.09)** | 150 | (20,2±1.36)** | 53 | (15.1±2.16)** | 16 | (31.1±4.40)** | 15 | (17.3±3.83)** |
| | 24 weeks | 208 | (24.9±1.31)** | 126 | (25.1±74)** | 52 | (22.5±2.37)** | 16 | (38.7±3.02)** | 14 | 16.4±5.09)** |
| | 48 weeks | 174 | (27.9±1.56)** | 101 | (29.0±205)** | 45 | (22.8±3.07)** | 15 | (36.2±5.25)** | 13 | (17.6±4.74)** |
| | 72 weeks | 140 | (25.8±1.54)** | 79 | (27.8±2.15)** | 39 | (22.2±2.57)** | 9 | (37.4±6.31)** | 13 | (16.5±3.44)** |
| | 96 weeks | 130 | (29)±)68)** | 73 | (32.1±2.46)** | 37 | (24.7±2.36)** | 7 | (31.2±9.44)* | 13 | (23.2±4.00)** |
| Total Cholesterol | Baseline | 269 | 276.0±2 1 | 185 | 273.0±2.4 | 53 | 285.2±4.9 | 16 | 282.0±10.6 | 15 | 272.8±9.1 |
| (mg/dL) | 12 weeks | 234 | (-7.2±0.63)** | 150 | (-74±0.76)** | 53 | (-6.9±1.50)** | 16 | (-6.3±2.23)** | 15 | (-7.0±2.47)* |
| | 24 weeks | 208 | (-9.2±0.6)** | 126 | (-7.8±0.88)** | 52 | (-11.8±1.92)** | 16 | (-8.2±2.20)** | 14 | (-13.7±1.96)** |
| | 48 weeks | 174 | (-14.0±0.87)** | 101 | (-10.5±0.94)** | 45 | (-23.1±1.77)** | 15 | (-11.3±2.82)** | 13 | (-13.4±2.42)** |
| | 72 weeks | 140 | (-15.7±0.97)** | 79 | (-11.4±1.15)** | 39 | (-24.1±1.41)** | 9 | (-13.9±4.51)** | 13 | (-18.2±2.99)** |
| | 96 weeks | 130 | (-15.5±1.10)** | 73 | (-10.0±1.15)** | 37 | (-23..8±2.05)** | 7 | (-15.1±2.62)** | 13 | (-23.1±3.45)** |
| Total # of Patients | | | 269 | | 185 | | 53 | | 16 | | 65 |
| Triglycerides | Baseline | 269 | 157.7±4.1 | 185 | 155.9±5.0 | 53 | 172.2±9.3 | 16 | 161.5±17.7 | 15 | 124.8±11.0 |
| (mg/dL) | 12 weeks | 234 | (-211±1.)80** | 150 | (-20.8±2.32)** | 53 | (-23.4±3.78)** | 16 | (-19.3±6.32)** | 15 | (-18.9±6.24)** |
| | 24 weeks | 208 | (-21.7±2.13)** | 126 | (-20.9±2.65)** | 52 | (-23.5±4.34)** | 16 | (-20.3±7.98)* | 14 | (-23.8±10.4)* |
| | 48 weeks | 174 | (-24.2±2.50)** | 101 | (-23.5±2.91)** | 45 | (-30.4±6.05)** | 15 | (-12.7±9.78) | 13 | (-22.2±6.27)** |
| | 72 weeks | 140 | (-28.1±2.75)** | 79 | (-26.5±3.67)** | 39 | (-37.4±3.53)** | 9 | (-1.6±20.7) | 13 | (-28.7±5.71)** |
| | 96 weeks | 130 | (-25.8±2.86)** | 73 | (-26.4±3.80)** | 37 | (32.2±4.10)** | 7 | (5.13±21.4) | 13 | (-20.5±8.32)* |

**LIPID RESULTS** Mean Percentage Change from Baseline LONG TERM POPULATION

| | | n | Subgroup Total | | Niaspan® Only | | Niaspan® + HmgCoA | | Niaspan® +BAS | | Niaspan® +Both |
|---|---|---|---|---|---|---|---|---|---|---|---|
| VLDL (mg/dL) | Baseline | 269 | 31.6±08 | 185 | 31.2±1.0 | 53 | 34.4±1.9 | 16 | 32.4±3.5 | 15 | 25.0±22 |
| | 12 weeks | 234 | (-21.2± .80)** | 150 | (-20.6±235)** | 53 | (-23.83.61)** | 16 | (-20.0±6.23)** | 15 | (-18.7±6.3)** |
| | 24 weeks | 208 | (-21.7±1.80)** | 126 | (-20.9±2.69)** | 52 | (1.23.7±4.30)** | 16 | (-20.4±7.98)* | 14 | (-23.9±10.4)* |
| | 48 weeks | 174 | (-24.0±2.58)** | 101 | (-23.4±2.91)** | 45 | (-29.4±6.57)** | 15 | (-13.3±9.80) | 13 | (-22.5±6.35)** |
| | 72 weeks | 140 | (-27.2±3.14)** | 79 | (-26.2±3.65)** | 39 | (-37.4±3.53)** | 9 | (10.5±31.0) | 13 | (-28.6±5.70)** |
| | 96 weeks | 130 | (-25.8±2.86)** | 73 | (-25.8±3.85)** | 37 | (-320±412)** | 7 | (-1.7±22.4) | 13 | (-20.6±8.09)* |
| TC to HDL Ratio | Baseline | 269 | 6.63±0.09 | 185 | 6.52±0.10 | 53 | 6.99±0.23 | 16 | 6.96±0.51 | 15 | 6.40±0.34 |
| | 12 weeks | 234 | (-21.0±0.88)** | 150 | (-21.6±.08)** | 53 | (-18.0±1.81)** | 16 | (-27.1±3.26)** | 15 | (-19.4±3.87)** |
| | 24 weeks | 208 | (-25.9±0.99)** | 126 | (-24.8±1.21)** | 52 | (-26.7±2.25)** | 16 | (-33.4±2.48)** | 14 | (-24.0±4.03)** |
| | 48 weeks | 174 | (-31.3±1.13)** | 101 | (-29.1±1.32)** | 45 | (-375±2.37)** | 15 | (-33.0±4.18)** | 13 | (-24.5±,1.61)** |
| | 72 weeks | 140 | (-31.8±1.15)** | 79 | (-29.4±1.51)** | 39 | (-36.9±1.87)** | 9 | (-36.0±5.32)** | 13 | (-28.7±4.21)** |
| | 96 weeks | 130 | (-33.4±1.16)** | 73 | (-30.1±1.64)** | 37 | (-38.4±1.82)** | 7 | (-33.5±3.85)** | 13 | (-37.2±3.10)** |
| LDL to HDL Ratio | Baseline | 269 | 4.85±0.08 | 185 | 4.75±0.08 | 53 | 5.12±0.20 | 16 | 5.16±0.47 | 15 | 4.80±0.31 |
| | 12 weeks | 234 | (-23.7±1.04)** | 150 | (-24.7±1.29)** | 53 | (-92±2.12)** | 16 | (-31.2±3.94)** | 15 | (-21.8±4.50)** |
| | 24 weeks | 208 | (-29.9±1.14)** | 126 | (-29.0±1.42)** | 52 | (-29.8±2.59)** | 16 | (-38.2±3.06)** | 14 | (-28.0±3.94)** |
| | 48 weeks | 173 | (-36.8±1.30)** | 100 | (-34.5±1.58)** | 15 | (-43.6±2.54)** | 15 | (-38.7±4.61)** | 13 | (-28.6±5.44)** |
| | 72 weeks | 140 | (-37.6±1.132)** | 79 | (-34.3±1.77)** | 39 | (-42.9±2.08)** | 9 | (-49.4±2.66)** | 13 | (-33.5±5.19)** |
| | 96 weeks | 130 | (-39.8±1.37)** | 73 | (-35.8±1.92)** | 37 | (-45.0±2.22)** | 7 | (-44.3±3.62)** | 13 | (-45.3±3.80)** |
| Total # of Patients | | | 269 | | 185 | | 53 | | 16 | | 15 | |
| Apolipopritein B (mg/dL) | Baseline | 244 | 148.1±1.23 | 165 | 145.7±1.41 | 48 | 155.8±2.85 | 16 | 149.3±-6.03 | 15 | 149.2±4.53 |
| | 12 weeks | 138 | (-9.8±.1.02)** | 76 | (-11.5±1.38)** | 37 | (-6.2±.2.06)** | 13 | (-11.4±2.90)** | 12 | (-8.0±2.82)** |
| | 24 weeks | 133 | (-14.0±11.06)** | 71 | (-12.6±1.37)** | 36 | (-16.1±2.56)** | 14 | (-13.72.28)** | 12 | (-16.6±2.48)** |
| | 48 weeks | 123 | (-18.7±1.14)** | 70 | (-15.4±1.33)** | 30 | (-26.2±2.60)** | 11 | (-19.2±3.14) | 12 | (-18.5±3.44)** |
| | 72 weeks | 43 | (-20.2±1.85)** | 31 | (-16.0±1.95)** | 11 | (-32.2±2.22)** | 0 | --- | 1 | (-19.3) |
| | 96 weeks | 0 | --- | 0 | --- | 0 | --- | 0 | --- | 0 | --- |
| l.p(a) | Baseline | 244 | 36.7±2.22 | 165 | 36.0±2.64 | 48 | 34.4±5.29 | 16 | 49.6±8.95 | 15 | 38.5±9.33 |

EP 1 017 390 B1

(continued)

**LIPID RESULTS** Mean Percentage Change from Baseline LONG TERM POPULATION

| | | n | Subgroup Total | | Niaspan® Only | | Niaspan® + HmgCoA | | Niaspan® +BAS | | Niaspan® +Both |
|---|---|---|---|---|---|---|---|---|---|---|---|
| (mg/dL) | 12 weeks | 139 | (-27.5±2.19)** | 78 | (-28.8±2.69)** | 36 | (-28.7±4.44)** | 13 | (-28.2±6.01)** | 12 | (-14.9±10.9) |
| | 24 weeks | 133 | (-28.7±2.29)** | 72 | (-30.4±2.70)** | 35 | (-28.0±5.31)** | 14 | (-29.8±5.99)** | 12 | (-19.5±10.0) |
| | 48 weeks | 131 | (-29.3±4.66)** | 74 | (-36.3±2.73)** | 32 | (-19.2±17.6)** | 12 | (-24.0±5.85)** | 13 | (-19.0±7.01)* |
| | 72 weeks | 44 | (-33.0±3.84)** | 29 | (-31.5±1.84)** | 12 | (-38.4±7.41)** | 0 | --- | 3 | (-24.7±12.6) |
| | 96 weeks | 0 | -- | 0 | --- | 0 | --- | 0 | --- | 0 | --- |

Note: Observed Values ± S.E. (percentage change ±S.E.)

Note: The --- indicates that no measurements have been collected for that visit. Observed Values ± S.E. (percent change ±S.E). Individual data are provided on ZIP diskette.

* Significant at $p \leq 0\ 05$, ** significant at $p$ a 0.01; matched-pair test

Table XII reports clinical chemistry parameters (liver function) for all 124 patients, who took concomitantly, once per day at night before going to bed. Niaspan® and an HMG-CoA reductase inhibitor. Table XII further reports clinical chemistry parameters (liver function) for 22 patients, who took concomitantly, once per day at night before going to bed, Niaspan® and BAS (a bile acid sequestrant, i.e., cholestyramine or colestipol). Table XII also reports clinical chemistry parameters (liver function) for 17 patients who took concomitantly, once per day at night before going to bed, Niaspan®, BAS (a bile acid sequestrant, i.e., cholestyramine or colestipol), and an HMG-CoA reductase inhibitor.

**TABLE XII**

CHEMISTRY PARAMETERS MEAN PERCENT CHANGE FROM BASELINE LONG TERM POPULATION

| | | LT Total | Niaspan Only | Niaspan & HMG-CoA | Niaspan & BAS | Niaspan & Both |
|---|---|---|---|---|---|---|
| | | n Mean±S.E. | n Mean±S.E. | n Mean ±S.E. | n Mean: ± S.E. | n Mean ± S.E. |
| Total Patients | | 617 | 454 | 124 | 22 | 17 |
| AST (mIU/mL) | | | | | | |
| | Baseline | 617 18.9±0.22 | 454 18.7±0.26 | 124 19.1±0.14 | 22 20.5±1.09 | 17 20.4±1.26 |
| | 12 weeks | 312 13.5±1.54)** | 352 (13.6±1.72)** | 121 (11.3±2.78)** | 22 (25.9±16.89) | 17 (11.4±4.35) * |
| | 48 weeks | 376 18.8±2.18)** | 240 (17.9±3.00)** | 101 (21.5±3.53)** | 20 (17.8±5.07) ** | 15 (16.6±9.14) |
| | 96 weeks | 133 (15.4±2.96)** | 76 (13.8±4.89) ** * | 37 (18.2±3.36)** | 7 (9.2±3.67)** | 13 (19.6±3.16) ** |
| ALT (mIU/mL) | | | | | | |
| | Baseline | 617 23.5±0.39 | 454 23.4±048 | 124 24.3±0.73 | 22 23.0±2.08 | 17 19.4±1.66 |
| | 12 weeks | 513 (1.8±1.51) | 353 (2.6±1.84) | 121 (-0.9±2.66) | 22 (6.7±11.47) | 17 (-1.3±5.70) |
| | 48 weeks | 376 (5 7±22)) ** | 240 (3.9±2.82) | 101 (89±4.26)* | 20 (3.6±5.74) | 15 (16.8±12.50) |
| | 96 weeks | 132 (5.7±3.50) | 75 (1.0±5.41 | 37 (10.9±5.09)* | 7 (-0.2±8.19) | 13 (21.4±6.49) |
| Alk>Ph. (mIU/mL) | Baseline | 617 699±0.70 | 454 66.0±0.83 | 124 67.0±1.56 | 22 59,2±2.30 | 17 63.6±3.29 |
| | 12 weeks | 513 (-0.3±0 55) | 353 (-0.6±0.70) | 121 (-0.8±0.93) | 22 (4.8±2.64) | 17 (1.3±3.36) |
| | 48 weeks | 375 (08±0.79) | 239 (08±100) | 101 (-0.4±1.19) | 20 (1.9±5.28) | 15 (7.8±4.87) |
| | 96 weeks | 132 (1.6±1.20) | 75 (2.5±171) | 37 (-2.0±1.43) | 7 (3.2±4.19) | 13 (6.2±5.10) |
| LDH (mIU/ML) | | | | | | |
| | Baseline | 617 147.90±0.92 | 454 147.3±1.04 | 124 148.4±1.96 | 22 152.3±7.03 | 17 156.5±6.80 |
| | 12 weeks | 513 (9.1±0.54)** | 353 (99±0.66) ** | 121 (8.2±1.07)** | 22 (14.0±3.23) ** | 17 (10.8±2.27) ** |
| | 48 weeks | 326 (15.2±0.76)** | 240 (14.5±0.93)** | 101 (16.1±1.59)** | 20 (21.6±3.63) ** | 15 (12.8±2.40) ** |
| | 96 weeks | 133 (17.9±1.04)** | 76 (17.1±1.23) ** | 37 (20.4±2.51)** | 7 (16.8±2.68) ** | 13 (16.1±2.97) ** |
| Total | | | | | | |

(continued)

CHEMISTRY PARAMETERS MEAN PERCENT CHANGE FROM BASELINE LONG TERM POPULATION

| | | LT Total | Niaspan Only | Niaspan & HMG-CoA | Niaspan & BAS | Niaspan & Both |
|---|---|---|---|---|---|---|
| | | n Mean±S.E. | n Mean±S.E. | n Mean ±S.E. | n Mean: ± S.E. | n Mean ± S.E. |
| Bili (mg/dL) | Baseline | 617 0.54±0.010 | 454 0.53±0.011 | 124 0.59±0.022 | 22 0.65±0.069 | 17 0.53±0.039 |
| | 12 weeks | 512 (1.6±1.33) | 352 (1.5±1.67) | 121 (-0.2±2.46) | 22 (1.3±4.84) | 17 (17.1±7.03)* |
| | 48 weeks | 376 (9.3+1.81)** | 240 (7.6±2.26)** | 101 (10.5±3.17)** | 20 (13.1±8.84) | 15 (24.3±12.55) |
| | 96 week | 132 (15.1±3.22)** | 75 (6.0±4.22) | 37 (25.5±5.20)** | 7 (26.3±11.49) | 13 (32.2±12.31)* |
| Dir. Bili. (mg/dL) | Baseline | 617 0.12±0.002 | 454 0.12±0.002 | 124 0.12±0.004 | 22 0.13±0.013 | 17 0.10±0.007 |
| | 12 weeks | 513 (8.7 ± 1.47) ** | 353 (8.1±1.76)** | 121 (8.7±3.06)** | 22 (10.2±6.50) | 17 (18.0±8.62) |
| | 48 weeks | 376 (26.4±6.66)** | 240 (26.6±10.24)** | 101 (24.4±4.23)** | 20 (24.1±7.68)** | 15 (40.1±12.24)** |
| | 96 wecks | 132 (27.2±3.52)** | 75 (17.8±4.22)** | 37 (40.6±6.77)** | 7 (16.7±7.66) | 13 (49.0±14.36)** |
| Amylase (mg/dl.) | Baseline | 617 51.2±0.78 | 454 51.2±0.92 | 124 52.2±1.72 | 22 49.3±3.70 | 17 45.6±3.20 |
| | 12 weeks | 513 (6.4±0.84)** | 353 (7 2±1.07)** | 121 (4.9±1.59)** | 22 (4.1±2.19) | 17 (3.4±3.39) |
| | 48 weeks | 376 (8.6±1.07)** | 240 (8.7±1.34)** | 101 (8.6±1.90)** | 20 (7.2±5.23) | 15 (10.1±7.06) |
| | 96 weeks | 132 (5.3±2.00)** | 75 (5.4±2.32)* | 37 (6.6±5.23) | 7 (3.1±3.58) | 13 (2.6±3.55) |

Note: Values are based upon two different central laboratories; thus, the Baseline observed value is presented for descriptive purposes only.

* Significant at $p \le 0.05$ ** Significant at $p \le 0.01$ Matched-pair t-test.

** Observed Value ±S E for Baseline (mean percent change from Baseline ± S E for Weeks 12, 48 and 96).

In Tables X-XII, positive numbers reflect percentage increases and negative numbers reflect percentage decreases.

Table XIII reports the number of patients and the % of the total patients in the study that recorded elevations above upper limits of normal (ULN) for selected clinical chemistry parameters. More particularly, Table XIII reports the number of patients and the % of the 124 patients, who took concomitantly, once per day at night before going to bed, Niaspan® and an HMG-CoA reductase inhibitor, that recorded elevations above ULN for selected clinical chemistry parameters. Table XIII further reports the number of patients and the % of the 22 patients, who took concomitantly, once per day at night before going to bed, Niaspan® and BAS (a bile acid sequestrant, i.e., cholestyramine or colestipol), that recorded elevations above ULN for selected clinical chemistry parameters. Table XIII further reports the number of patients and the % of the 17 patients, who took concomitantly, once per day at night before going to bed, Niaspan®. BAS (a bile acid sequestrant, i.e., cholestyramine or colestipol), and an HMG-CoA reductase inhibitor, that recorded elevations above ULN for selected clinical chemistry parameters.

**TABLE XIII**
**TREATMENT EMERGENT ABNORMALITIES IN SELECTED CHEMISTRY PARAMETERS LONG TERM POPULATION**

| | | LT Total[1] | Niaspan® Only | Niaspan®& HMG-CoA | Niaspan® & BAS | Niaspan® & Both |
|---|---|---|---|---|---|---|
| Total Patients | | 617 | 454 | 124 | 22 | 17 |
| AST | >Normal | 70(11%) | 44(10%) | 17(14%) | 5(23%) | 4(24%) |
| (mIU/mL) | >1.3xULN | 28(5%) | 17(4%) | 6(5%) | 3(14%) | 2(12%) |
| | >2xULN | 5(1%) | 3(<1%) | 1(<1%) | 1(6%) | 0 |
| | >3xULN | 1(<1%) | 1(<1%) | 0 | 0 | 0 |
| ALT | >Normal | 44(7%) | 23(5%) | 14 (11%) | 2(9%) | 5(29%) |
| (mIU/mL) | >1.3xULN | 15(2%) | 4(<1%) | 5(4%) | 2(9%) | 4(24%) |
| | >2xULN | 3(<1%) | 1(<1%) | 2(2%) | 0 | 0 |
| | >3xULN | 1(<1%) | 0 | 1(<1%) | 0 | 0 |
| Alk. Phos. | >Normal | 17(3%) | 9(2%) | 3(2%) | 2(9%) | 3(18%) |
| (mIU/mL) | >1.3xULN | 3(1%) | 0 | 2(2%) | 1(5%) | 0 |
| LDH | >Normal | 94(15%) | 60(13%) | 23(19%) | 8(36%) | 3(18%) |
| (mIU/mL) | >1.3xULN | 6(<1%) | 4(<1%) | 1(<1%) | 0 | 1(6%) |
| Fasting Glue | >Normal | 111(18%) | 67(15%) | 36(28%) | 4(18%) | (24%) |
| (mg/dL) | >1.3xULN | 6(<1%) | 3(<1%) | 3(2/%) | 0 | 0 |
| Uric Acid | >Normal | 89(14%) | 49(11%) | 28(23%) | 7(32%) | 5(29%) |
| (mIU/mL) | >1.3xULN | 5(<t%) | 3(<)% | (401%) | 0 | 1( 6%) |
| Total Bili | >Normal | 10(2%) | 5(1%) | 4(3%) | 0 | (6%) |
| (mg/dL) | >1x3.ULN | 2(<1%) | 1(<1%) | 1(<1%) | 0 | 0 |
| Amylase | >Normal | 18(3%) | 11(2%) | 7(6%) | 0 | 0 |
| (mg/dL) | >1.3xULN | 6(<1%) | 5(1%) | 1(<1%) | 0 | 0 |
| | >2xULN | 1(<1%) | (<1%) | 0 | 0 | 0 |
| Phosphorus | <Normal | 159(26%) | 96(21%) | 47(38%) | 9(41%) | 7(41%) |
| (mg/dL) | <2.0mg/dL | 19(3%) | 14( 3%) | 4(3%) | 1(5%) | 0 |

Note: Percentages are calculated from the total number of patients in each column.
Abnormal liver test results for Patient 3512 are not included in this table as the data were collected at a local hospital.
Refer to the initial Safety Update (Vol 1, ¶ 12-13.37)

Table XIV reports the number of patients and the % of the total patients in the study that recorded elevations 2 or 3 times above upper limits of normal (ULN) for the AST and ALT clinical chemistry parameters. More particularly, Table XIV reports the number of patients and the % of the 124 patients, who took concomitantly, once per day at night before going to bed. Niaspan® and an HMG-CoA reductase inhibitor, that recorded elevations which were 2 or 3 times above ULN for the AST and ALT clinical chemistry parameters. Table XIV is consistent with that reported in Table XIII.

**TABLE XIV**
**LONG-TERM STUDY SAFETY DATA LIVER FUNCTION TESTS**

| | Niaspan® N=454* | Niaspan® + HMG-CoA Reductase Inhibitor N=124** |
|---|---|---|
| AST > 2xULN | 3 (<1%) | 1(<1%) |
| AST > 3xULN | 1 (<1%) | 0 |

(continued)

**LONG-TERM STUDY SAFETY DATA LIVER FUNCTION TESTS**

| | Niaspan® N=454* | Niaspan® + HMG-CoA Reductase Inhibitor N=124** |
|---|---|---|
| ALT>2xULN | 1 (< 1 %) | 2 (1.6%) |
| ALT > 3xULN | 0 | 1(<1%) |
| *Mean follow-up approximately 52 weeks | | |
| **Mean follow-up approximately 43 weeks | | |

The data reported in Tables XI-XIV evidences that a pharmaceutical combination of the present invention, e.g., sustained release nicotinic acid and an immediate release HMG-CoA reductase inhibitor, given concomitantly, once per day at night before bedtime, is effective in reducing serum lipid levels, and in particular total cholesterol, VLDL-cholesterol, LDL-cholesterol, triglycerides, apolipoprotein B and Lp(a) levels, and is effective in reducing the total cholesterol to HDL-cholesterol ratio and the LDL-cholesterol to HDL-cholesterol ratio. The data reported in Tables XI-XIV also evidences that a pharmaceutical combination of the present invention, e.g.. sustained release nicotinic acid and an immediate release HMG-CoA reductase inhibitor, given concomitantly, once per day at night before bedtime, is effective in enhancing or increasing HDL-cholesterol levels. Also, it is believed that the data reported in Tables XI-XIV evidences that a pharmaceutical combination of the present invention, e.g., sustained release nicotinic acid and an immediate release HMG-CoA reductase inhibitor, given concomitantly, once per day at night before bedtime, is more effective in reducing LDL-cholesterol levels than when either sustained release nicotinic acid or an immediate release HMG-CoA reductase inhibitor are given in similar dosages once per day at night before going to bed, but alone. Still further, it is believed that the data reported in Tables XI-XIV evidences that a pharmaceutical combination of the present invention, e.g., sustained release nicotinic acid and an immediate release HMG-CoA reductase inhibitor, given concomitantly, once per day at night before bedtime, is more effective in increasing HDL-cholesterol levels than when an immediate release HMG-CoA reductase inhibitor is given by itself in a similar dosage once per day at night before going to bed.

The data reported in Tables XI-XIV also evidences that such concomitant therapy, e.g., sustained release nicotinic acid and an immediate release HMG-CoA reductase inhibitor, given once per day at night before bedtime can be administered and the benefits achieved without inducing hepatotoxicity, myopathy or rhabdomyolysis, or at least without inducing in an appreciable number of individuals hepatotoxicity, myopathy or rhabdomyolysis to such a level that would require discontinuation of such therapy. Moreover, the data reported in Table XII evidences that such concomitant therapy, e.g., sustained release nicotinic acid and an immediate release HMG-CoA reductase inhibitor, given once per day at night before bedtime can be administered and the benefits achieved without adversely effecting glucose metabolism or uric acid levels, or without adversely effecting in at least an appreciable number of individuals glucose metabolism or uric acid levels to such an extent that discontinuation of such therapy would be required.

**Claims**

1. A coated tablet for oral administration to alter lipids in an Individual without causing drug-induced hepatotoxiaty, myopathy or rhabdomyotysis, said coated tablet comprising a lipid-altering amount of nicotinic acid or a compound metabolized to nicotinic add by the body, selected from nicotinyl alcohol tartrate, d-glucitol hexanicotinate, aluminium nicotinate, niceritrol, d,l-alpha-tocopheryl nicotinate, 6-OH-nicotinic acid, nicotinaria acid, nicotinamide, nicotinamide-N-Oxide, 6-OH-nicotinamide, NAD, N-methyl-2-pyrrydine-8-carboxamide, N-methyl-nicotinamide, N-ribosyl-2-pyridone-5-carboxide, N-methyl-4-pyridone-5-carboxamide, bradilian, sorbinicate, hexanicite, ronitol, and methyl, ethyl, propyl or butyl alcohol esters of nicotinic acid or combinations thereof, in an extended release form, and a coating containing a lipid-altering amount of an HMG-CoA reductase inhibitor in an immediate release form.

2. The coated tablet of claim 1, wherein said HMG-CoA reductase inhibitor is selected from atorvastatin, cerivastatin, flavastatin, lovastatin, pravastatin and simvastatin.

3. The coated tablet of claim 1, wherein said coated tablet is oval, flat or oval, convexed in shape.

4. The coated tablet of claim 1, wherein said coated tablet is round, flat or round, convexed in shape.

5. The coated tablet of claim 1, wherein said coated tablet is capsule-shaped.

6. The coated tablet of claim 1, wherein said coated tablet is coated with an enteric coating.

7. The coated tablet of claim 1, wherein said coated tablet includes a flush inhibiting agent to reduce the capacity of the nicotinic acid or the compound metabolized to nicotinic acid by the body to provoke a flushing reaction in a subject.

8. The coated tablet of claim 7, wherein said flush inhibiting agent is a nonsteroidal anti-inflamrnatory.

9. The coated tablet of claim 8 non-steroidal anti-inflammatory, wherein said is selected from indomethacin, sulindac, etodolac, aspirin, salicylate salts, ibuprofen, fluribprofen, fenoprophen, suprofen, benoxaprofen, ketoprofen, carprofen, naproxen, sodium naproxen, aclofenac, diclofenac, fenclofenac, tolmactin, zomepirac, meclofenamate, mefanamic add, oxyphenbutazone, phenylbutazone and piroxicam.

10. The coated tablet of claim 1, said coated tablet further including a lipid-altering agent selected from a bile acid sequestrant, an N-substituted ethanolamine derivative, an azulene derivative, a disubstituted urea derivative, an ionene, a poly(diallylmethylamine) derivative, an omega-3-fatty acid and a fibric acid.

11. The coated tablet of claim 1, said coated tablet further including a lipid-altering agent selected from cholestyramine, colestipol, DEAESephadex, probucol, lipostabil, Elsat E5050 (an N-substituted ethanolamine derivative), imanixil (HOE-402) tatr;ahydralipstafin (THL), isitigmastanylphosphorylcholine aminocyctodextrin, Ajinomoto AJ-814 (azulene derivative), melinamide, neomycin, quarternary amine poly(diallydimethylammonium chloride), gemfibrozil, clofibrate, bexafibrate, fenofibrate, ciprofibrate and clinofibrate.

12. The coated tablet of claim 1, said coated tablet further Including chotestyramine.

13. The coated tablet of claim 1, said coated tablet further including colestipol.

14. The coated tablet as claimed in any of claims 1 to 13 wherein the coating is selected from carboxymethylcellulose sodium, camuba wax, cellulose acetate phthalate, cetyl alcohol, confectioner's sugar, ethylcellulose, gelatine, hydroxyethyl cellulose, hydroxyl propyl cellulose, hydroxyl propyl methyl cellulose, liquid glucose, maltodextrin, methyl cellulose, microcrystalline wax, polymethacrylates, polyvinyl alcohol, shellac, sucrose, talc, titanium dioxide and/or zein.

15. The coated tablet as claimed in claim 6 wherein the enteric coating is selected from cellulose acetate phthalate, polyvinylacetate phthalate, hydroxypropyl methylcellulose phthalate and/or methacrylic acid-methacrylate copolymers.

16. The coated tablet as claimed in any of claims 1 to 15, wherein said coated tablet is administered orally once per day during the evening hours or before or at bedtime.

**Patentansprüche**

1. Manteltablette zur oralen Verabreichung, um Lipide in einem Individuum zu ändern, ohne Arzneistoff-induzierte Hepatotoxizität, Myopathie oder Rhabdomyolyse zu verursachen, wobei die Manteltablette eine lipidändernde Menge Nikotinsäure oder eine Verbindung, die vom Körper zu Nikotinsäure metabolisiert wird, ausgewählt aus Nikotinylalkoholtartrat, d-Glucitolhexanikotinat, Aluminiumnikotinat, Niceritrol, d,l-$\alpha$-Tocopherylnikotinat, 6-OH-Nikotinsäure, Nikotinariasäure (nicotinaria acid), Nikotinamid, Nikotinamid-N-oxid, 6-OH-Nikotinamid, NAD, N-Methyl-2-pyrridin-8-carboxamid, N-Methyl-Nikotinamid, N-Ribosyl-2-pyridon-5-carboxid, N-Methyl-4-pyridon-5-carboxamid, Bradilian, Sorbinicat, Hexanicit, Ronitol und Methyl-, Ethyl-, Propyl- oder Butylalkoholestern der Nikotinsäure oder Kombinationen davon, in verzögert freigesetzter Form, und einen Mantel, umfassend eine lipidändernde Menge eines HMG-CoA-Reduktaseinhibitors zur sofortigen Freisetzung, umfasst.

2. Manteltablette nach Anspruch 1, wobei der HMG-CoA-Reduktaseinhibitor ausgewählt ist aus Atorvastatin, Cerivastatin, Flavastatin, Lovastatin, Pravastatin und Simvastatin.

3. Manteltablette nach Anspruch 1, wobei die Manteltablette eine ovale, flache, oder ovale, konvexe Form aufweist.

4. Manteltablette nach Anspruch 1, wobei die Manteltablette eine runde, flache, oder runde, konvexe Form aufweist.

5. Manteltablette nach Anspruch 1, wobei die Manteltablette kapselförmig ist.

**6.** Manteltablette nach Anspruch 1, wobei die Manteltablette mit einer magensaftresistenten Beschichtung überzogen ist.

**7.** Manteltablette nach Anspruch 1, wobei die Manteltablette ein Flush inhibierendes Mittel einschließt, um die Fähigkeit der Nikotinsäure oder der Verbindung, die vom Körper zu Nikotinsäure metabolisiert wird, eine Flush-Reaktion im Patienten hervorzurufen, zu verringern.

**8.** Manteltablette nach Anspruch 7, wobei das Flush inhibierende Mittel ein nichtsteroidales Antiphlogistikum ist.

**9.** Manteltablette nach Anspruch 8, wobei das nichtsteroidale Antiphlogistikum ausgewählt ist aus Indomethacin, Sulindac, Etodolac, Aspirin, Salizylatsalzen, Ibuprofen, Fluribprofen, Fenoprophen, Suprofen, Benoxaprofen, Ketoprofen, Carprofen, Naproxen, Natriumnaproxen, Aclofenac, Diclofenac, Fenclofenac, Tolmectin, Zomepirac, Meclofenamat, Mefanaminsäure, Oxyphenbutazon, Phenylbutazon und Piroxicam.

**10.** Manteltablette nach Anspruch 1, wobei die Manteltablette ferner ein lipidänderndes Mittel einschließt, ausgewählt aus einem die Gallensäureabsonderung auslösenden Mittel, einem N-substituierten Ethanolaminderivat, einem Azulenderivat, einem disubstituierten Harnstoffderivat, einem Ionen, einem Poly(diallylmethylamin)derivat, einer w-3-Fettsäure und einer Fibrinsäure.

**11.** Manteltablette nach Anspruch 1, wobei die Manteltablette ferner ein lipidänderndes Mittel einschließt, ausgewählt aus Cholestyramin, Colestipol, DEAE-Sephadex, Probucol, Lipostabil, Eisai E5050 (ein N-substituiertes Ethanolaminderivat), Imanixil (HOE-402) Tetrahydrolipstatin (THL), Isitigmastanylphosphorylcholin, Aminocyclodextrin, Ajinomoto AJ-814 (Azulenderivat), Melinamid, Neomycin, Quartäraminpoly(diallyldimethylammoniumchlorid), Gemfibrozil, Clofibrat, Bexafibrat, Fenofibrat, Ciprofibrat und Clinofibrat.

**12.** Manteltablette nach Anspruch 1, wobei die Manteltablette ferner Cholestyramin einschließt.

**13.** Manteltablette nach Anspruch 1, wobei die Manteltablette ferner Colestipol einschließt.

**14.** Manteltablette nach einem der Ansprüche 1 bis 13, wobei die Beschichtung ausgewählt ist aus Carboxymethylcellulosenatrium, Karnaubawachs, Celluloseacetatphthalat, Cetylalkohol, Puderzucker, Ethylcellulose, Gelatine, Hydroxyethylcellulose, Hydroxypropylcellulose, Hydroxypropylmethylcellulose, flüssige Glukose, Maltodextrin, Methylcellulose, mikrokristallines Wachs, Polymethacrylate, Polyvinylalkohol, Schellack, Saccharose, Talkum, Titandioxid und/oder Zein.

**15.** Manteltablette nach Anspruch 6, wobei die magensaftresistente Beschichtung ausgewählt ist aus Celluloseacetatphthalat, Polyvinylacetatphthalat, Hydroxypropylmethylcellulosephthalat und/oder Methacrylsäure-Methacrylat-Copolymeren.

**16.** Manteltablette nach einem der Ansprüche 1 bis 15, wobei die Manteltablette einmal pro Tag während der Abendstunden oder vor der oder zur Schlafenszeit oral verabreicht wird.

**Revendications**

**1.** Comprimé enrobé pour une administration par voie orale en vue d'altérer des lipides chez un individu sans provoquer d'hépatotoxicité, de myopathie ou de rhabdomyolyse induite par un médicament, ledit comprimé enrobé comprenant une quantité d'acide nicotinique à effet d'altération des lipides, ou un composé métabolisé en acide nicotinique par le corps, choisi parmi le tartrate d'alcool de nicotinyle, l'hexanicotinate de d-glucitol, le nicotinate d'aluminium, le nicéritrol, le nicotinate de d,1-alpha-tocophéryle, l'acide 6-OH-nicotinique, l'acide nicotinaria, le nicotinamide, le N-oxyde de nicotinamide, le 6-OH-nicotinamide, le NAD, le N-méthyl-2-pyrridine-8-carboxamide, le N-méthyl-nicotinamide, le N-ribosyl-2-pyridone-5-carboxyde, le N-méthyl-4-pyridone-5-carboxamide, le bradilian, le sorbinicate, l'hexanicite, le ronitol et les esters d'acide nicotinique avec les alcools méthylique, éthylique, propylique ou butylique, ou leurs combinaisons, sous une forme à libération prolongée, et un enrobage contenant une quantité exerçant un effet d'altération des lipides d'un inhibiteur de l'HMG-CoA réductase, sous une forme à libération immédiate.

**2.** Comprimé enrobé selon la revendication 1, dans lequel ledit inhibiteur de l'HMG-CoA réductase est choisi parmi l'atorvastatine, la cérivastatine, la fluvastatine, la lovastatine, la pravastatine et la simvastatine.

3.  Comprimé enrobé selon la revendication 1, dans lequel ledit comprimé enrobé est de forme ovale et plate ou de forme ovale et convexe.

4.  Comprimé enrobé selon la revendication 1, dans lequel ledit comprimé enrobé est de forme ronde et plate ou de forme ronde et convexe.

5.  Comprimé enrobé selon la revendication 1, dans lequel ledit comprimé enrobé se présente sous la forme d'une capsule.

6.  Comprimé enrobé selon la revendication 1, dans lequel ledit comprimé enrobé est revêtu d'un enrobage entérique.

7.  Comprimé enrobé selon la revendication 1, dans lequel ledit comprimé enrobé comprend un agent inhibiteur de rougeur pour réduire la capacité de l'acide nicotinique, ou du composé métabolisé en acide nicotinique par le corps, de provoquer une réaction de rougeur chez le sujet.

8.  Comprimé enrobé selon la revendication 7, dans lequel ledit agent inhibiteur de rougeur est un anti-inflammatoire non stéroïdien.

9.  Comprimé enrobé selon la revendication 8, dans lequel ledit anti-inflammatoire non stéroïdien est choisi parmi l'indométhacine, le sulindac, l'étodolac, l'aspirine, le salicylate, l'ibuprofène, le flurbiprofène, le fénoprofène, le suprofène, le bénoxaprofène, le kétoprofène, le carprofène, le naproxène, le naproxène de sodium, l'aclofénac, le diclofénac, le fenclofénac, la tolmetine, le zomépirac, le méclofénamate, l'acide méfénamique, l'oxyphénbutazone, la phénylbutazone et le piroxicam.

10. Comprimé enrobé selon la revendication 1, ledit comprimé enrobé comprenant en outre un agent d'altération des lipides, choisi parmi un séquestrant d'acide biliaire, un dérivé d'éthanolamine N-substituée, un dérivé d'azulène, un dérivé d'urée disubstituée, un ionène, un dérivé de poly(diallylméthylamine), un acide gras de type oméga-3 et l'acide fibrique.

11. Comprimé enrobé selon la revendication 1, ledit comprimé enrobé comprenant, en outre, un agent d'altération des lipides choisi parmi la cholestyramine, le colestipol, le DEAE-Sephadex, le probucol, le lipostabil, le composé Eisai E5050 (un dérivé d'éthanolamine N-substituée), l'imanixil (HOE-402), la tétrahydrolipstatine (THL), l'isitigmastanylphosphorylcholine, l'amino-cyclodextrine, le composé Ajinomoto AJ-814 (un dérivé de l'azulène), le mélinamide, la néomycine, le poly(chlorure de diallydiméthylammonium) d'amine quaternaire, le gemfibrozil, le clofibrate, le bexafibrate, le fénofibrate, le ciprofibrate et le clinofibrate.

12. Comprimé enrobé selon la revendication 1, ledit comprimé enrobé comprenant, en outre, de la cholestyramine.

13. Comprimé enrobé selon la revendication 1, ledit comprimé enrobé comprenant également du colestipol.

14. Comprimé enrobé selon l'une quelconque des revendications 1 à 13, dans lequel l'enrobage est choisi parmi la carboxyméthylcellulose de sodium, la cire de Carnauba, l'acétate-phtalate de cellulose, l'alcool cétylique, le sucre en poudre, l'éthylcellulose, la gélatine, l'hydroxyéthylcellulose, l'hydroxypropylcellulose, l'hydroxypropylméthylcellulose, le glucose liquide, la maltodextrine, la méthylcellulose, la cire microcristalline, des polyméthacrylates, l'alcool polyvinylique, la gomme-laque, le saccharose, le talc, le dioxyde de titane et/ou la zéine.

15. Comprimé enrobé selon la revendication 6, dans lequel l'enrobage entérique est choisi parmi l'acétate-phtalate de cellulose, le poly(acétate-phtalate de vinyle), le phtalate d'hydroxypropylméthylcellulose et/ou des copolymères acide méthacrylique-méthacrylate.

16. Comprimé enrobé selon l'une quelconque des revendications 1 à 15, dans lequel ledit comprimé enrobé est administré par voie orale une fois par jour aux horaires de soirée, avant celles-ci ou à l'heure du coucher.